# FASCICULE DE BREVET EUROPEEN

(11) **EP 2 442 783 B1**
(45) Date de publication et mention de la délivrance du brevet: **09.08.2017**
(21) Numéro de dépôt: 10734772.6
(22) Date de dépôt: 17.06.2010
(51) Int. Cl.: A61K 8/35, A61L 9/14, C07C 49/207, C07C 49/217

(54) **NOUVELLES COMPOSITIONS DÉSODORISANTES ET PRODUITS DÉSODORISANTS LES RENFERMANT**
NEUE DESODORIERENDE ZUSAMMENSETZUNGEN UND DESODORIERENDE PRODUKTE DAMIT
NOVEL DEODORISING COMPOSITIONS AND DEODORISING PRODUCTS CONTAINING SAME

(30) Priorité: 18.06.2009 EP 09007987
(43) Date de publication de la demande: 25.04.2012
(73) Titulaire: Robertet S.A., 06130 Grasse (FR)
(72) Inventeur: CASAZZA, André, 06130 Grasse (FR); KERVERDO, Raymond, 06460 St. Vallier De Thiey (FR); ZIEGLER, Lydia, 06130 Grasse (FR); BREVARD, Hugues, 06130 Grasse (FR)
(74) Mandataire: Santarelli
(86) Numéro de dépôt international: PCT/FR2010/000448
(87) Numéro de publication internationale: WO 2010/146258

(56) Documents cités:
- EP-A1- 1 224 947
- WO-A1-2006/076821
- WO-A2-2008/149102
- DE-A1- 4 417 752
- NL-A- 8 104 274
- US-A- 3 325 367
- US-A- 3 852 355
- US-A- 3 944 621
- US-A- 5 968 795
- DATABASE WPI Week 199136 Thomson Scientific, London, GB; AN 1991-262306 XP002604917, -& JP 3 170450 A (HASEGAWA CO LTD) 24 juillet 1991 (1991-07-24)
- DATABASE CA [Online] CHEMICAL ABSTRACTS SERVICE, COLUMBUS, OHIO, US; EL-SAYED, SAID ABBAS MOHAMED: "Studies on nitrification inhibition with many acetylenic compounds in soils", XP002634591, extrait de STN Database accession no. 2004:273961 & EL-SAYED, SAID ABBAS MOHAMED: "Studies on nitrification inhibition with many acetylenic compounds in soils", 2003, ALEXANDRIA SCIENCE EXCHANGE , 24(3), 327-339 CODEN: ALSEEF; ISSN: 1110-0176
- DATABASE CA [Online] CHEMICAL ABSTRACTS SERVICE, COLUMBUS, OHIO, US; ROE, R. MICHAEL ET AL: "A novel geminal diol as a highly specific and stable in vivo inhibitor of insect juvenile hormone esterase", XP002634592, extrait de STN Database accession no. 1997:649074 & ROE, R. MICHAEL ET AL: "A novel geminal diol as a highly specific and stable in vivo inhibitor of insect juvenile hormone esterase", 1997, ARCHIVES OF INSECT BIOCHEMISTRY AND PHYSIOLOGY , 36(3), 165-179 CODEN: AIBPEA; ISSN: 0739-4462
- DATABASE CA [Online] CHEMICAL ABSTRACTS SERVICE, COLUMBUS, OHIO, US; GLAZUNOVA, N. P. ET AL: "Antimicrobial activity of acetylene keto ethers", XP002634593, extrait de STN Database accession no. 1974:413316 & GLAZUNOVA, N. P. ET AL: "Antimicrobial activity of acetylene keto ethers", 1974, KHIMIKO-FARMATSEVTICHESKII ZHURNAL , 8(4), 18-23 CODEN: KHFZAN; ISSN: 0023-1134
- DATABASE CA [Online] CHEMICAL ABSTRACTS SERVICE, COLUMBUS, OHIO, US; YAMADA, TAKESHI ET AL: "Characteristic of Artemisia capillaris extract and development with", XP002634594, extrait de STN Database accession no. 2006:455159 & YAMADA, TAKESHI ET AL: "Characteristic of Artemisia capillaris extract and development with", 2006, FRAGRANCE JOURNAL , 34(4), 60-67 CODEN: FUJAD7; ISSN: 0288-9803
- DATABASE CA [Online] CHEMICAL ABSTRACTS SERVICE, COLUMBUS, OHIO, US; GAVRILOV, L. D. ET AL: "Synthesis of diarylpropynones and their antibiotic activity", XP002634595, extrait de STN Database accession no. 1979:22498 & GAVRILOV, L. D. ET AL: "Synthesis of diarylpropynones and their antibiotic activity", 1978, KHIMIKO-FARMATSEVTICHESKII ZHURNAL , 12(9), 42-5 CODEN: KHFZAN; ISSN: 0023-1134
- DATABASE CA [Online] CHEMICAL ABSTRACTS SERVICE, COLUMBUS, OHIO, US; OSHIMA, SATOSHI ET AL: "Capillin-containing odorless antibacterial and antifungal agents, and their compositions", XP002634596, extrait de STN Database accession no. 2003:673775 & JP 2003 238310 A (SAKAMOTO YAKUHIN KOGYO CO., LTD., JAPAN) 27 août 2003 (2003-08-27)
- DATABASE CA [Online] CHEMICAL ABSTRACTS SERVICE, COLUMBUS, OHIO, US; "Acetylene derivatives as nematocides", XP002634597, extrait de STN Database accession no. 1982:16086 & JP 56 123904 A (KANESHO CO., LTD., JAPAN) 29 septembre 1981 (1981-09-29)
- DATABASE CA [Online] CHEMICAL ABSTRACTS SERVICE, COLUMBUS, OHIO, US; "Soil disinfectant composition", XP002634598, extrait de STN Database accession no. 1982:117584 & JP 56 154402 A (KANESHO CO., LTD., JAPAN) 30 novembre 1981 (1981-11-30)
- DATABASE CA [Online] CHEMICAL ABSTRACTS SERVICE, COLUMBUS, OHIO, US; HILLERS, S. ET AL: "Antibiotic activity of some acetylene derivatives of furan", XP002634599, extrait de STN Database accession no. 1967:471302 & HILLERS, S. ET AL: "Antibiotic activity of some acetylene derivatives of furan", 1967, LATVIJAS PSR ZINATNU AKADEMIJAS VESTIS , (6), 104-8 CODEN: LZAVAL; ISSN: 0132-6422
- DATABASE CA [Online] CHEMICAL ABSTRACTS SERVICE, COLUMBUS, OHIO, US; HILLERS, S. ET AL: "Fungistatic and bacteriostatic activity of some acetylenic derivatives of pathogenic fungi", XP002634600, extrait de STN Database accession no. 1969:459997 & HILLERS, S. ET AL: "Fungistatic and bacteriostatic activity of some acetylenic derivatives of pathogenic fungi", 1968, KHIM. ATSETILENA , 427-30. EDITOR(S): SHOSTAKOVSKII, M. F. PUBLISHER: IZD. "NAUKA", MOSCOW, USSR. CODEN: 20WJAZ
- DATABASE CA [Online] CHEMICAL ABSTRACTS SERVICE, COLUMBUS, OHIO, US; KATSUTA, YOSHIO: "Preservation of wood", XP002634601, extrait de STN Database accession no. 1986:408202 & JP 60 257202 A (DAINIPPON JOCHUGIKU CO., LTD., JAPAN) 19 décembre 1985 (1985-12-19)
- DATABASE CA [Online] CHEMICAL ABSTRACTS SERVICE, COLUMBUS, OHIO, US; KATSUTA, SUMIO: "Pyrethrin insecticides", XP002634602, extrait de STN Database accession no. 1974:823 & JP 48 003366 B 30 janvier 1973 (1973-01-30)
- DATABASE WPI Week 198617 Thomson Scientific, London, GB; AN 1986-110338 XP002634855, -& JP 61 053238 A (KAO CORP) 17 mars 1986 (1986-03-17)

## Description

La présente demande concerne de nouvelles compositions désodorisantes renfermant au moins un composé de la famille des cétones acétyléniques et les produits désodorisants les renfermant.

La lutte contre les mauvaises odeurs a conduit à utiliser de nombreuses substances de grande variété, par exemple les substances phénoliques, des huiles essentielles, des résines, des aldéhydes ou des cétones, des dérivés alcooliques, des esters ou autres.

On a notamment utilisé divers aldéhydes, seuls ou en mélange avec de nombreux autres produits. Bien que donnant satisfaction, ces produits ne présentent pas toujours l'efficacité désirée.

Il existe donc toujours un besoin en composition désodorisante plus efficaces et éventuellement d'une odeur agréable.

Après de longs travaux, la Demanderesse a découvert que des molécules de la famille des cétones acétyléniques peuvent être utilisées dans des compositions désodorisantes, particulièrement du fait de leur propriété destructrice des molécules malodorantes. On comprend donc que dans le présent texte l'utilisation de l'expression "composition désodorisante" se réfère à une composition dont l'effet désodorisant est lié aux propriétés de destruction physique des molécules malodorantes par les cétones acétyléniques et non un quelconque masquage de la mauvaise odeur.

Par la suite dans le présent texte, l'expression "cétone acétylénique" est utilisée pour désigner des molécules chimiques comprenant au moins un enchaînement céto-α-acétylénique (-CO-C≡C- ou -C≡C-CO-).

Ainsi l'invention a pour objet premier la destruction physique de molécules malodorantes par une composition désodorisante caractérisée en ce qu'elle comprend au moins un composé de la famille des cétones acétyléniques répondant à la formule générale 1

**R-(CO)ₖ-C≡C-(A)-(C≡C)ₘ-(CO)ₙ-R₁** **(1)**

dans laquelle
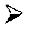 R et R₁ peuvent représenter, indépendamment ou simultanément, un radical choisi parmi
   ∘ une chaîne alkyle comprenant de 1 à 9, préférentiellement de 3 à 7 atomes de carbone, linéaire ou ramifiée, substituée ou non, ou
   ∘ un cycloalcane comprenant de 3 à 8, préférentiellement de 5 ou 6 atomes de carbone, substitué ou non ; ou
   ∘ un cycle furanique, saturé ou non, substitué ou non ; ou
   ∘ un cycle pyranique, saturé ou non, substitué ou non ; ou
   ∘ un cycle aromatique comprenant de 6 à 8 atomes de carbone, substitué ou non ; ou encore
   ∘ un atome d'hydrogène.
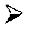 A représente un groupement choisi parmi -(CH₂)ₓ avec x représentant un entier de valeur 0 à 6, préférentiellement de 0 à 4, (-CO)ₗ avec l représentant un entier de valeur 0 ou 1, ou encore un enchaînement - (CH₂)_{y}-O-(CH₂)_{z}- avec y et z représentant, simultanément ou indépendamment, un entier de valeur 0 à 6, préférentiellement de 0 à 4, étant entendu que x + y ne dépasse pas la valeur 6 ;
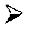 k, m et n sont, simultanément ou indépendamment, un entier de valeur 0 ou 1, étant entendu que k et n ne peuvent être simultanément de valeur 0 si l est égal à 0.
à l'exception de la 2-methyl-4-nonyn-3-one de formule C₄H₉-C≡C-CO-CH(CH₃)₂.

Selon l'invention on entend par chaîne alkyle comprenant de 1 à 9 atomes de carbone, un radical méthyle, éthyle, propyle, butyle, pentyle, hexyle, heptyle, octyle, nonyle, linéaire ou ramifié, substitué ou non, en particulier par un ou des groupement(s) oxo, sans toutefois que le total d'atomes de carbone dans la molécule n'excède 18, préférentiellement 16.

Selon encore l'invention on entend par un cycloalcane comprenant de 3 à 8 atomes de carbone, un radical cyclopropyle, cyclobutyle, cyclopentyle, cyclohexyle, cycloheptyle, cyclooctyle, cyclopropylcarbonyle, cyclobutylcarbonyle, cyclopropylméthyle ou encore cyclobutylméthyle.

Selon aussi l'invention on entend par un cycle aromatique comprenant de 6 à 8 atomes de carbone, un radical choisi parmi le phényle, benzyle ou phénétyle, éventuellement substitué par un ou deux substituants, identiques ou différents, choisis parmi les substituants méthyle, diméthyle, méthoxy, diméthoxy, hydroxy, acétyle, cyano.

Selon l'invention, les radicaux R et R₁ peuvent être, simultanément ou indépendamment substitués par un ou plusieurs radicaux choisis parmi les radicaux méthyle, éthyle, propyle, butyle ou encore un radical 2-oxopropyle.

Avantageusement selon l'invention, R et R1 peuvent être préférentiellement un radical acétonyle, pentyle, hexyle, heptyle, cyclopropyle, cyclopentyle , cyclohexyle, furyle ou phényle, anisyle, dimethoxyphényle, tolyle et R₁ peut être un atome d'hydrogène.

Selon l'invention, le composé de formule 1 peut être dans la composition en une quantité comprise entre 0,01% et 40%, préférentiellement entre 0,1% et 15%, encore plus préférentiellement de 1% à 10% en poids total de la composition.

Selon l'invention, la composition peut comprendre un ou plusieurs composés répondant à la formule 1.

Selon l'invention, on pourra utiliser préférentiellement dans les compositions au moins un des composés choisi parmi la :
- 1-octyn-3-one de formule O₅H₁₁-CO-C≡CH ;
- 3-octyn-2-one de formule C₄H₉-C≡C-CO-CH₃ ;
- 4-octyn 3-one de formule C₃H₇-C≡C-CO-C₂H₅ ;
- 4-nonyn-3-one de formule C₃H₇-CH₂-C≡C-CO-C₂H₅ ;
- 2-nonyn-4-one de formule CH₃-C≡C-CO-C₅H₁₁ ;
- 5-nonyn-4-one de formule C₃H₇-CO-C≡C-CH₂-C₂H₅ ;
- 1-decyn-3-one de formule C₇H₁₅-CO-C≡CH ;
- 4-decyn-3-one de formule C₅H₁₁-C≡C-CO-C₂H₅;
- 6-decyn-5-one de formule C₃H₇-C≡C-CO-C₄H₉ ;
- 5-decyn-4-one de formule C₄H₉-C≡C-CO-C₃H₇ ;
- 2-decen-5-yn-4-one de formule C₄H₉-C≡C-CO-CH=CH-CH₃ ;
- 1-(p-methoxyphenyl)-2-propyn-1-one de formule H₃C-O-C₆H₄-CO-C≡CH ;
- 1-cyclopropyl-2-heptyn-1-one de formule C₄H₉-C≡C-CO-C₃H₅ (cyclopropyl);
- 2-methyl-5-decyn-4-one de formule C₄H₉-C≡C-CO-CH₂-CH(CH₃)₂ ;
- 9-methyl-5-decyn-4-one de formule C₃H₇-CO-C≡C-CH₂-CH₂-CH(CH₃)₂ ;
- 2-methyl-4-octyn-3-one de formule C₃H₇-C≡C-CO-CH(CH₃)₂ ;
- 1-pheny4-2-propyn-1-one de formule C₆H₅-CO-C≡CH ;
- 4-phenyl-3-butyn-2-one de formule C₆H₅-C≡C-CO-CH₃ ;
- 5-phenyl-4-pentyn-3-one de formule C₆H₅-C≡C-CO-C₂H₅ ;
- 6-phenyl-5-hexyn-2,4-dione de formule C₆H₅-C≡C-CO-CH₂-CO-CH₃ ;
- 1-phenyl-1-heptyn-3-one de formule C₆H₅-C≡C-CO-C₄H₉ ;
- 1-(2-furyl)-3-phenyl-2-propyn-1-one de formule C₆H₅-C≡C-CO-C₄H₃O (furyl) ;
- 1-(2-furyl)-2-octyn-1-one de formule C₅H₁₁-C≡C-CO-C₄H₃O (furyl) ;
- C₆H₅-C≡C-CO-C₄H₃O (furyl) ;
- 3-cyclopropyl-1-(p-tolyl)-2-propyn-1-one de formule C₃H₂-C≡C-CO-C₆H₄-CH₃ ;
- 1-cyclopropyl-4-methyl-1-hexyn-3-one de formule C₃H₅-C≡C-CO-CH(C₂H₅)-CH₃ (cyclopropyl) ;
- 9-hexadecyn-8-one de formule C₆H₁₃-C≡C-CO-C₇H₁₅ ;
- 5-ethyl-11-methyl-7-dodecyn-6-one de formule (CH₃)₂CH-C₂H₄-C≡C-CO-CH(C₂H₅)-C₄H₉ ;
- 7-tetradecyn-6-one de formule C₆H₁₃-C≡C-CO-C₅H₁₁ ;
- 1-cyclohexyl-4-ethy4-1-hexyn-3-one de formule C₆H₁₁-C≡C-CO-CH(C₂H₅)₂ (cyclohexyl) ;
- 1-cyclopentyl-4-nonyn-3-one de formule C₄H₉-C≡C-CO-C₂H₄-C₅H₉ (cyclopentyl) ;
- 1-cyclohexyl-2-heptyn-1-one de formule C₄H₉-C≡C-CO-C₆H₁₁ (cyclohexyl) ;
- 1-(p-tolyl)-2-butyn-1-one de formule CH₃-C≡C-CO-C₆H₄-(p-CH₃) ;
- 2,2,8-trimethyl-4-nonyn-3-one de formule (CH₃)₂CH-C₂H₄-C≡C-CO-C(CH₃)₃
- 2,2-dimethyl-4-hexyn-3-one de formule CH₃-C≡C-CO-C(CH₃)₃ ;
- 1,4-diphenyl-2-butyn-1,4-dione de formule C₆H₅-CO-C≡C-CO-C₆H₅ ;
- 5-decyn-2,4-dione de formule C₄H₉-C≡C-CO-CH₂-CO-CH₃ ;
- 1-(3,4-dimethoxyphenyl)-2-butyn-1-one de formule CH₃-C≡C-CO-C₆H₃-(OCH₃)₂ ;
- 2,5-heptadiyn-4-one de formule H₃C-C≡C-CO-C≡C-CH₃ ;
- 3-hexyn-2,5-dione de formule CH₃-CO-C≡C-CO-CH₃ ;
- 4-octyn-3,6-dione de formule C₂H₅-CO-C≡C-CO-C₂H₅ ;
- 2,7-dimethyl-4-octyn-3,6-dione de formule (CH₃)₂CH-CO-C≡C-CO-CH(CH₃)₂ ;
- 2,7-diméthyl-4-octyn-3-one de formule (CH₃)₂CH-CH₂-C≡C-CO-CH(CH₃)₂;
- 4,7-undecadiyn-6-one de formule C₃H₇-C≡C-CO-C≡C-C₃H₇ ;
- 5-decyn-4,7-dione de formule C₃H₇-CO-C≡C-CO-C₃H₇ ;
- 2,9-dimethyl-5-decyn-4,7-dione de formule (CH₃)₂CH-CH₂-CO-C≡C-CO-CH₂-CH(CH₃)₂ ;
- 6-dodecyn-5,8-dione de formule C₄H₉-CO-C≡C-CO-C₄H₉ ;
- 2,11-dimethyl-6-dodecyn-5,8-dione de formule (CH₃)₂CH-C₂H₄-CO-C≡C-CO-(CH₂)₂-CH(CH₃) ;
- 2,9-dimethyl-5-decyn-4-one de formule (CH₃)₂CH-C₂H₄-C≡C-CO-CH₂-CH(CH₃)₂ ;
- 7-tetradecyn-6,9-dione de formule C₅H₁₁-CO-C≡C-CO-C₅H₁₁ ;
- 4,10-undecadiyn-3-one de formule HC≡C-C₄H₈-C≡C-CO-C₂H₅ ;
- 2,12-dimethyl-7-oxa-4,9-tridecadiyn-3,11-dione de formule (CH₃)₂-CO-C≡C-CH₂-O-CH₂-C≡C-CO-CH(CH₃)₂, ou encore
- 4,10-tetradecadiyn-3,12-dione de formule C₂H₅-CO-C≡C-C₄H₈-C≡C-CO-C₂H₅.
Très préférentiellement on pourra utiliser au moins un composé choisi parmi
- la 1-phenyl-2-propyn-1-one de formule C₆H₅-CO-C≡CH ;
- la 1-decyn-3-one de formule C₇H₁₅-CO-C≡CH ;
- la 1-(2-furyl)-2-octyn-1-one de formule C₅H₁₁-C≡C-CO-C₄H₃O (furyl) ;
- la 1-(2-furyl)-3-phenyl-2-propyn-1-one de formule C₆H₅-C≡C-CO-C₄H₃O (furyl) ;
- la 1-octyn-3-one de formule C₅H₁₁-CO-C≡CH ;
- la 1-(p-tolyl)-2-butyn-1-one de formule CH₃-C≡C-CO-C₆H₄-(p-CH₃) ;
- la 2,5-heptadiyn-4-one de formule CH₃-C≡C-CO-C≡C-CH₃ ;
- la 1-(p-methoxyphenyl)-2-propyn-1-one de formule CH₃O-C₆H₄-CO-C≡CH ;
- la 1,4-diphenyl-2-butyn-1,4-dione de formule C₆H₅-CO-C≡C-CO-C₆H₅ ; ou encore
- la 4,7-undecadiyn-6-one de formule C₃H₇-C≡C-CO-C≡C-C₃H₇.

Selon une variante de l'invention, on pourra utiliser dans les compositions toute combinaison associant au moins 2 des composés entrant dans la formule 1, préférentiellement ceux précédemment listés et encore plus préférentiellement les couples formés de 2 composés choisi parmi les couples :
- 1-phenyl-2-propyn-1-one / 1-decyn-3-one ;
- 1-(2-furyl)-2-octyn-1-one / 1-decyn-3-one ;
- 1-(2-furyl)-3-phenyl-2-propyn-1-one / 1-decyn-3-one ;
- 1-octyn-3-one /1-decyn-3-one ;
- 1-(p-tolyl)-2-butyn-1-one /1-decyn-3-one ;
- 2,5-heptadiyn-4-one /1-decyn-3-one ;
- 1-(p-methoxyphenyl)-2-propyn-1-one / 1-decyn-3-one ;
- 1,4-diphenyl-2-butyn-1,4-dione / 1-decyn-3-one ;
- 4,7-undecadiyn-6-one / 1-decyn-3-one ;
- 1-(2-furyl)-2-octyn-1-one / 1-phenyl-2-propyn-1-one ;
- 1-(2-furyl)-3-phenyl-2-propyn-1-one / 1-phenyl-2-propyn-1-one ;
- 1-octyn-3-one / 1-phenyl-2-propyn-1-one ;
- 1-(p-tolyl)-2-butyn-1-one / 1-phenyl-2-propyn-1-one ;
- 2,5-heptadiyn-4-one / 1-phenyl-2-propyn-1-one ;
- 1-(p-methoxyphenyl)-2-propyn-1-one / 1-phenyl-2-propyn-1-one ;
- 1,4-diphenyl-2-butyn-1,4-dione / 1-phenyl-2-propyn-1-one ;
- 4,7-undecadiyn-6-one / 1-phenyl-2-propyn-1-one ;
- 1-(2-furyl)-3-phenyl-2-propyn-1-one / 1-(2-furyl)-2-octyn-1-one ;
- 1-octyn-3-one/ 1-(2-furyl)-2-octyn-1-one 1-(p-tolyl)-2-butyn-1-one ;
- 2,5-heptadiyn-4-one / 1-(2-furyl)-2-octyn-1-one ;
- 1-(p-methoxyphenyl)-2-propyn-1-one / 1-(2-furyl)-2-octyn-1-one ;
- 1,4-diphenyl-2-butyn-1,4-dione / 1-(2-furyl)-2-octyn-1-one ;
- 4,7-undecadiyn-6-one / 1-(2-furyl)-2-octyn-1-one ;
- 1-octyn-3-one / 1-(2-furyl)-3-phenyl-2-propyn-1-one ;
- 1-(p-tolyl)-2-butyn-1-one / 1-(2-furyl)-3-phenyl-2-propyn-1-one ;
- 2,5-heptadiyn-4-one / 1-(2-furyl)-3-phenyl-2-propyn-1-one ;
- 1-(p-methoxyphenyl)-2-propyn-1-one / 1-(2-furyl)-3-phenyl-2-propyn-1-one ;
- 1,4-diphenyl-2-butyn-1,4-dione / 1-(2-furyl)-3-phenyl-2-propyn-1-one ;
- 4,7-undecadiyn-6-one /1-(2-furyl)-3-phenyl-2-propyn-1-one ;
- 1-(p-toty4)-2-butyn-1-one / 1-octyn-3-one ;
- 2,5-heptadiyn-4-one / 1-octyn-3-one ;
- 1-(p-methoxyphenyl)-2-propyn-1-one / 1-octyn-3-one ;
- 1,4-diphenyl-2-butyn-1,4-dione / 1-octyn-3-one ;
- 4,7-undecadiyn-6-one / 1-octyn-3-one ;
- 2,5-heptadiyn-4-one / 1-(p-tolyl)-2-butyn-1-one ;
- 1-(p-methoxyphenyl)-2-propyn-1-one / 1-(p-toly()-2-butyn-1-one ;
- 1,4-diphenyl-2-butyn-1,4-dione / 1-(p-tolyl)-2-butyn-1-one ;
- 4,7-undecadiyn-6-one / 1-(p-tolyl)-2-butyn-1-one ;
- 1-(p-methoxyphenyl)-2-propyn-1-one / 2,5-heptadiyn-4-one ;
- 1,4-diphenyl-2-butyn-1,4-dione / 2,5-heptadiyn-4-one ;
- 4,7-undecadiyn-6-one / 2,5-heptadiyn-4-one ;
- 1,4-diphenyl-2-butyn-1,4-dione / 1-(p-methoxyphenyl)-2-propyn-1-one ;
- 4,7-undecadiyn-6-one / 1-(p-methoxyphenyl)-2-propyn-1-one ;
- 4,7-undecadiyn-6-one / 1,4-diphenyl-2-butyn-1,4-dione.

Selon l'invention, le composé de formule 1 peut être dans la composition présent en une quantité comprise entre 0,1% et 40%, préférentiellement entre 0,1% et 10%, en poids total de la composition.

Les composés de formule 1 sont connus pour leur grande réactivité avec les thiols et les amines.

Ils sont aisément obtenus par les voies de synthèse habituelles en chimie et particulièrement
- soit par action d'un chlorure d'acide sur un dérivé sodé d'un alcyne selon

   R-COCl + NaC≡C-R₁ → R-CO-C≡C-R₁
- soit par oxidation d'un précurseur alcool secondaire selon

   R-CH(OH)-C≡C-R₁ → R-CO-C≡C-R₁
- soit par oxidation directe d'un alcyne selon

   R-CH₂-C≡C-CH₂-R₁ → R-CO-C≡C-CH₂-R₁/R-CH₂-C≡C-CO-R₁

Selon une variante de l'invention, la composition désodorisante peut en outre comprendre au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique. Dans cette variante, la composition peut comprendre un mélange de deux ou plusieurs aldéhydes.

Selon une autre variante de l'invention, préférée, la composition désodorisante peut comprendre en outre un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique. Dans cette variante, la composition peut comprendre un mélange d'au moins un premier et d'au moins un second aldéhyde. Mais bien évidement le premier aldéhyde peut être un mélange de deux ou plusieurs aldéhydes de classe A et le second aldéhyde peut être un mélange de deux ou plusieurs aldéhydes choisis parmi ceux de classe B.

Selon l'invention, les aldéhydes de classe A ou B peuvent être choisis parmi les aldéhydes naturels ou de synthèse, particulièrement parmi ceux classés non dangereux pour l'environnement selon la directive Européenne "Substances dangereuses", autorisés en alimentaire (n°FEMA) et en parfumerie par l'IFRA.

Selon l'invention, l'aldéhyde aliphatique acyclique et non terpénique de classe A peut être choisi parmi
- le decanal ;
- l'undecanal ;
- le dodecanal ;
- le 10-undecenal ;
- le 2-methyl undecanal ;
- le trimethyl-2,6,10-undecen-9-al (ADOXAL™) ; ou encore
- le tetramethyl-2,3,5,5-hexanal.

Encore selon l'invention, l'aldéhyde alicyclique non terpénique de classe A peut être choisi parmi
- le 2,4-dimethylcyclohex-3-en-1-carboxaldehyde,
- le 1,2-dimethylcyclohex-3-en-1-carboxaldehyde,
- le 3,5-dimethylcyc(ohex-3-en-1-carboxaldehyde,
- le 2,4,6-trimethylcyclohex-3-en-1-carboxaldehyde (ISOCYCLOCITRAL™),
- le tricyclo[5.2.1.0(0.6)]decyliden-8)-4-butanal (DUPICAL™) ;
- le trimethyl-2,6,10-undecen-9 al (ADOXAL™) ;
- le (methyl-4-penten-3-yl)-4-cyclohex-3-en-3-yl-1-carboxaldehyde ;
- le 6-methyl-8-(1-methylethyl)bicyclo[2.2.2]-5-octen-2-carboxaldehyde (MACEAL™) ; ou encore
- le 8,8-dimethyl-2,3,4,5,6,7-hexahydro-1H-naphthalene-2-carboxaldehyde (ALDEHYDE 111™) ;
- le 5-methyl furfural ;
- le 1-methyl-4-(4-methylpentyl)cyclohex-3-en-1-carboxaldehyde, ;
- le 3-(5-methyl-2-furyl)butanal,6,6-dimethylbicyclo[3.1.1]-2-heptenyl-2-propanal ;
- le tricyclodecanylcarboxaldehyde ; ou encore
- le butylcinnamaldehyde

Préférentiellement l'aldéhyde alicyclique non terpénique (AliNT) de classe A pourra être choisi parmi
- le 8,8-dimethyl-2,3,4,5,6,7-hexahydro-1H-naphthalene-2 carboxaldehyde (ALDEHYDE 111™) ;
- le 5-methyl furfural ;
- le 1-methyl-4-(4-methylpentyl)cyclohex-3-en-1-carboxaldehyde ;
- le 3-(5-methy)-2-furyl)butana),6,6-dimethylbicyclo[3.1.1]-2-heptenyl-2-propanal ;
- le tricyclodecanylcarboxaldehyde ; ou encore
- le butylcinnamaldehyde.

Encore selon l'invention, l'aldéhyde terpénique (Terp) de classe A peut être choisi parmi
- le citronellal ;
- le 7-hydroxy-3,7-dimethyloctanal (hydroxycitronellal) ;
- le 2,2,3-trimethyl-3-cyclopenten-1-acetaldehyde (α-campholenaldehyde) ;
- le p-menth-1-en-9-al (carvomenthenal) ;
- le 7,7-dimethylbicyclo[3.1.1]hept-3-en-4-carboxaldehyde (myrtenal) ; ou encore
- le 2-isopropyl-5-methyl-2-hexenal (isodihydrolavandulal).

Préférentiellement selon l'invention, l'aldéhyde terpénique peut être choisi parmi
- le 2,2,3-trimethyl-3-cyclopenten-1-acetaldehyde (α-campholenaldehyde) ;
- le carvomenthenal ;
- le myrtenal ; ou encore
- l'isodihydrolavandulal.

Encore selon l'invention, l'aldéhyde aliphatique de classe A substitué par un groupe aromatique peut être choisi parmi
- le 2-methyl-3-(p-isopropylphenyl)propanal ;
- le 3-(4-tert-butylphenyl)butanal ;
- le phenylacetaldehyde ;
- le 3-phenylpropanal ;
- le 2-phenylpropanal ;
- le 2-methyl-3-(p-methylphenyl)propanal ;
- 2-methyl-3-(3,4-methylenedioxyphenyl)propanal (HELIONAL™);
- le cyclamenaldehyde ;
- le 2-methyl-3-(4-tert-butylphenyl)propanal (LILIAL™);
- le 3-(4-methoxyphenyl)-2-methylpropanal (CANTHOXAL™); ou encore
- le phenylacetaldehyde.

Préférentiellement selon l'invention, l'aldéhyde aliphatique substitué par un groupe aromatique peut être choisi parmi
- le 2-methyl-3-(p-isopropylphenyl)propanal ;
- le 3-(4-tert-butylphenyl)butanal ;
- le phenylacetaldehyde ;
- le 3-phenylpropanal ;
- le 2-phenylpropanal ; ou encore
- le 2-methyl-3-(p-methylphenyl)propanal.

Par "aldéhyde bifonctionnel", on entend selon l'invention les aldéhydes possédant par ailleurs une autre fonction comme par exemple une fonction éther-oxyde ou alcool. L'aldéhyde bifonctionnel peut être selon l'invention choisi parmi
- les alcoxy-acétaldéhydes ;
- les hydroxy-aldéhydes ;
- les alcoxy-aldéhydes ;
comme par exemple
- les 3 et 4-(4-hydroxy-4-methylpentyl)-3-cyclohexen-1-carboxaldehyde (LYRAL™) ;
- le methoxydicyclopentadiencarboxaldehyde ;
- le 3-(1,3-benzodioxol-5-yl)-2-methylpropanal ; ou encore
- le 7-hydroxy-3,7-dimethyloctanal (hydroxycitronellal).
Préférentiellement selon l'invention, l'aldéhyde bifonctionnel peut être
- le methoxydicyclopentadiencarboxaldehyde ; ou encore
- le 7-hydroxy-3,7-dimethyloctanal (hydroxycitronellal).

Selon l'invention, l'aldéhyde qui possède une insaturation de type non aromatique (classe B) portée par le carbone en position alpha de la fonction aldéhyde peut être choisi parmi
- le citral (néral et géranial) ;
- le myrtenal ;
- le perillaldehyde ;
- les furyl-2-carboxaldehydes substitués ou non, préférentiellement ceux non substitués ou substitués par au moins un méthyl ;
- le 2,4-heptadienal ;
- le 2E,4E-undecadienal ;
- le 2E,4E-dodecadienal ;
- le 2E-decenal ;
- le 2E,4Z,7Z-tridecatrienal ;
- le 2E,6Z-nonadienal ;
- le 2-furfurylidenebutanal ;
- le 3,7-dimethyl-2-methylen-6-octenal ; ou encore
- le 2E-dodecenal.

Préférentiellement selon l'invention, l'aldéhyde qui possède une insaturation de type non aromatique (classe B) portée par le carbone en position alpha de la fonction aldéhyde peut être
- le citral ;
- le myrtenal ;
- le perillaldehyde ;
- le 2E-dodecenal ; ou encore
- le 2-furfurylidenebutanal.

L'aldéhyde (de classe B) qui possède une insaturation éthylénique en alpha, conjuguée avec un cycle aromatique peut être choisi parmi
- le cinnamaldhyde ;
- l'α-amylcinnamaldehyde (JASMONAL™) ;
- l'α-hexylcinnamaldehyde ;
- le (E)-3-(2-methoxyphenyl)prop-2-enal ;
- le (E)-3-(4-methoxyphenyl)prop-2-enal ;
- l'α-methylcinnamaldehyde ;
- le 1-benzofurane-2-carboxaldehyde ;
- le 2-phenyl-2-butenal ; ou encore
- le 5-methyl-2-phenyl-2-hexenal.

Préférentiellement selon l'invention, l'aldéhyde qui possède une insaturation éthylénique en alpha, conjuguée avec un cycle aromatique peut être
- le (E)-3-(2-methoxyphenyl)prop-2-enal ;
- le (E)-3-(4-methoxyphenyl)prop-2-enal ;
- le 1-benzofurane-2-carboxaldehyde ;
- le 2-phenyl-2-butenal ; ou encore
- le 5-methyl-2-phenyl-2-hexenal.

L'aldéhyde porté par un cycle aromatique, lui-même éventuellement substitués par ailleurs, peut être choisi parmi
- la vanilline ;
- l'ethylvanilline;
- le vanillyl acetate ;
- le benzaldehyde ;
- l'anisaldehyde.
- le cinnamaldhyde ;
- l'α-amylcinnamaldehyde (JASMONAL™) ;
- l'α-hexylcinnamaldehyde ;
- le (E)-3-(2-methoxyphenyl)prop-2-enal ;
- le (E)-3-(4-methoxyphenyl)prop-2-enal ;
- l'α-methylcinnamaldehyde ;
- le 1-benzofurane-2-carboxaldehyde ;
- le 2-phenyl-2-butenal ; ou encore
- le 5-methyl-2-phenyl-2-hexenal.

Préférentiellement selon l'invention, porté par un cycle aromatique, lui-même éventuellement substitués par ailleurs, peut être choisi parmi
- la vanilline ;
- l'ethylvanilline ;
- le vanillyl acetate ;
- le benzaldehyde ; ou encore
- l'anisaldehyde.

Dans des conditions préférentielles de mise en oeuvre de la seconde variante de l'invention ci-dessus décrite, le premier aldéhyde peut être choisi de préférence parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde peut être choisi de préférence parmi les aldéhydes qui possèdent une insaturation de type non aromatique portée par le carbone alpha.

Tout particulièrement, le premier aldéhyde peut être choisi parmi les aldéhydes aliphatiques ou acycliques et non terpéniques et le second parmi les aldéhydes terpéniques possédant une insaturation sur le carbone alpha ci-dessus désignés.

Des couples d'aldéhydes particulièrement intéressants peuvent être choisis notamment parmi les couples suivants, sans limitation aucune :
- le dodecanal et le myrtenal ;
- le dodecanal et le citral ;
- l'adoxal et le perillaldehyde ;
- le triplal et le citral ;
- le maceal et le citral ;
- l'adoxal et le myrtenal ;
- le decanal et le citral ; ou encore
- le 10-undecenal et le myrtenal.

Selon la première variante de l'invention la composition peut comprendre l'aldéhyde en une quantité comprise entre 0,5% et 50% de la composition, préférentiellement en une quantité comprise entre 1% et 20% de la composition.

Selon la seconde variante de l'invention la composition peut comprendre les aldéhydes des classes A et B en proportions relatives l'un par rapport à l'autre en proportions de 80/20 à 20/80 notamment en proportions de 50/50 et dans une quantité totale d'aldéhydes comprise entre 1 % et 90 % de la composition, préférentiellement en une quantité comprise entre 10% et 30% de la composition.

Les compositions désodorisantes selon l'invention, en plus de leurs remarquables propriétés désodorisantes, possèdent en outre par elles-mêmes des propriétés odoriférantes éventuellement capables de remplacer les mauvaises odeurs par leur propre odeur.

Selon encore une autre variante de l'invention les compositions désodorisantes peuvent en outre comprendre un agent masquant.

Ces compositions peuvent donc comporter au moins un agent odoriférant tel que ceux habituellement utilisés en parfumerie comme des alcools, huiles essentielles, substances phénoliques, esters.

Ces compositions désodorisantes trouvent leur application dans toutes les conditions où la lutte contre les mauvaises odeurs est recherchée, quelle que soit leur origine.

Les compositions selon l'invention peuvent renfermer en outre des substances spécifiques, comme par exemple des bactéricides.

Ces compositions désodorisantes sont avantageusement formulées selon les techniques habituelles.

C'est pourquoi la présente demande a également pour objet les produits désodorisants caractérisés en ce qu'ils renferment les compositions ci-dessus décrites.

Ces produits peuvent se présenter sous forme de pulvérisateurs aérosols, supports solides imprégnés, liquides, crèmes, poudres, etc. Ils peuvent être réalisés selon les méthodes connues de l'homme de l'art.

L'invention a encore pour objet l'utilisation d'un composé de la famille des cétones acétyléniques répondant à la formule 1 dans une composition désodorisante, particulièrement dans des compositions désodorisantes comprenant au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique, et encore plus préférentiellement dans une composition désodorisante comprenant un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

D'autres avantages, buts et caractéristiques pourront ressortir de la description qui va suivre, faite, dans un but explicatif et nullement limitatif, des dessins annexés dans lesquels :
La figure 1 illustre la dégradation de la butylamine sous l'effet de cétones acétyléniques selon l'invention.
La figure 2 illustre la dégradation de la butylamine sous l'effet d'un agent neutralisant connu auquel est ajouté une cétone acétylénique selon l'invention.

### Partie expérimentale

Les exemples qui suivent illustrent la présente invention, sans toutefois la limiter.

### Préparation des cétones acétyléniques

### I- Cétones acétyléniques disubstituées R-CO-C≡C-R₁

### I.I Préparation de la 4-decyn-3-one (atmosphère inerte)

Dans un réacteur de 1 litre on charge 345 mL de diglyme (RN 111-96-6) et 17,2 g (748 mmoles) de sodium (RN 7440-23-5) coupé en morceaux. On chauffe à une température un peu supérieure à la fusion du sodium et, sous bonne agitation, on ajoute alors en quatre fois à 1 h d'intervalle 71,7 g (745 mmoles) soit 97,5 mL de 1-heptyne (RN 628-71-7) en solution dans 100 mL de diglyme. Le milieu s'épaissit pour former une suspension beige. On agite encore pendant 2 h 30 à la même température. On refroidit l'ensemble à 25-30°C et on le verse lentement sur une solution agitée de 64,8 g (700 mmoles) soit 61,0 mL de chlorure de propionyle (RN 79-03-8) dans 600 mL d'éther éthylique. Une réaction exothermique se produit. On agite ensuite pendant 1 h sans s'occuper de la température.

On lave par 4 x 1 litre d'eau, on sèche et concentre sous pression réduite (25 mm Hg) pour obtenir 98 g d'un liquide jaune orangé. Ce dernier est fractionné sous pression réduite (2 mm Hg). On recueille la fraction qui passe à 70-71°C. Rendement 50 g soit 47%.

De manière analogue sont préparées (exemples non limitatifs): la 5-decyn-4-one, la 3-octyn-2-one, la 4-phenyl-3-butyn-2-one, la 9-hexadecyn-8-one, la 1-(2-furyl)-3-phényl-2-propyne-1-one et la 1-cyclopentyl-4-nonyn-3-one.

### I.II Préparation du mélange de la 4-nonyn-3-one et 5-nonyn-4-one

Dans un réacteur de 500 mL on charge 13,8 g (111 mmoles) de 4-nonyne (RN 20184-91-2), 175 mL de tert-butanol (RN 75-65-0), 770 mg (4,5 mmoles) de chlorure cuivrique dihydrate (RN 10125-13-0), et 33,4 mL (244 mmoles) de tert-butyl hydropéroxyde à 70% (RN 75-91-2). Sous bonne agitation et atmosphère d'oxygène pur issu d'un réservoir gradué, on porte le milieu à 60-65°C. On constate au bout de trois heures que le milieu n'absorbe plus d'oxygène et a consommé la quantité théorique.

On verse sur de la glace et extrait trois fois à l'éther. Les phases organiques réunies sont lavées quatre fois à l'eau salée à 5%. Après séchage et concentration on obtient environ 40 g d'un liquide vert qu'on fractionne par distillation sous pression réduite (20 mm Hg). Les deux isomères, qu'on ne sépare pas, distillent ensemble entre 92 et 97°C.

De manière analogue on prépare la 2-methyl-5-decyn-4-one et la 9-methyl-5-decyn-4-one ainsi que la 1-phenyl-1-pentyn-3-one (cette dernière en un isomère unique, un seul site de la 1-phenyl-1-pentyne de départ pouvant être oxydé).

### II- Cétones acétyléniques vraies R-CO-C≡CH, cétones acétyléniques disubstituées R-CO-C≡C-R1

### IIA- Préparation de la 1-octyn-3-one

### IIA-1) Préparation de la 1-octyn-3-ol (atmosphère inerte)

Dans un réacteur de 750 mL on charge 40,1 g (400 mmoles) soit 49,2 mL d'hexanal (RN 66-25-1) et 400 mL de tétrahydrofurane anhydre et sous agitation on ajoute par portions 100 g d'une suspension d'acétylure de sodium (RN 1066-26-8) à 18% dans le xylène (375 mmoles). Une réaction exothermique se produit. On agite ensuite encore pendant 2 h sans s'occuper de la température. Après hydrolyse, extractions et concentration, on fractionne sous pression réduite.

Il est toutefois à noter qu'un meilleur résultat est obtenu par l'emploi de bromure d'éthynylmagnésium (RN 4301-14-8). Ainsi par exemple pour le 1-decyn-3-ol.

### IIA-2) Préparation de la 1-octyn-3-one

Dans un réacteur de 1500 mL on charge 25,24 g (200 mmoles) soit 29,1 mL de 1-octyn-3-ol, 600 mL d'acétone et 90 mL d'eau. Sous agitation énergique on ajoute en 1 h 30 une solution de 29,4 g (100 mmoles) de bichromate de potassium (RN 7778-50-9) dans 300 mL d'eau et 22 mL d'acide sulfurique. La coloration passe de l'orange au vert. A la fin, on agite encore pendant 30 mn puis on ajoute 1000 mL d'eau en 30 mn également.

Après extractions à l'éther éthylique, lavages, séchage et concentration sous pression faiblement réduite, on obtient 25 g d'un liquide orange qu'on fractionne sous pression réduite (20 mm Hg). On recueille la fraction qui passe à 74-76°C.

De manière analogue on prépare la 1-phenyl-2-propyn-1-one et la 1-decyn-3-one, la 1-(p-methoxyphenyl)-2-propyn-1-one.

### IIA-3 autres préparations

De manière analogue, en utilisant le sel d'un alcyne substitué (Na-C=C-R) on prépare la 1-cyclohexyl-4-ethyl-1-hexyn-3-one, la 7-tetradecyn-8-one, la 2-nonyn-4-one, 9-hexadecyn-8-one, 1-(p-tolyl)-2-butyn-1-one.

### III- Evaluation de l'effet des compositions selon l'invention sur les mauvaises odeurs

Des tests organoleptiques, avec des formules contenant ou non des cétones acétyléniques décrites précédemment relatent l'abaissement significatif de mauvaises odeurs complexes (odeurs de cuisine, toilettes, tabac).

### III-1 Protocole :

Le test olfactif est réalisé avec un panel expert de 25 personnes.

Les cétones acétyléniques à tester sont préparées à la concentration de 1 mol/L dans le dipropylen glycol.

La solution malodorante de référence est également préparée à la concentration 1 mol/L.

Un premier pilulier contient 1 g de la solution malodorante de référence.

Un mélange équimolaire de 1 g d'une solution de cétone acétylénique et de 1 g de solution de solution malodorante de référence est placé dans d'autres piluliers (1 pilulier par cétone acétylénique à tester).

La mauvaise odeur dégagée par le premier pilulier se voit attribuer la valeur de référence 10 (solution malodorante de référence).

A l'aide d'un carré latin (tableau indiquant l'ordre à suivre par chaque juge du panel de testeurs pour éviter les biais), les autres piluliers sont évalués en comparaison avec le premier pilulier.

Les résultats sont ensuite transformés en pourcentage de mauvaise odeur restante.

Les cétones acétyléniques suivantes sont testées sur différentes solutions de solution malodorantes de référence :
A : 6-phenyl-5-hexyn-2,4-dione de formule C₆H₅-C≡C-CO-CH₂-CO-CH₃ ;
B : 4-phenyl-3-butyn-2-one de formule C₆H₅-C≡C-CO-CH₃ ;
C : 2-decen-5-yn-4-one de formule C₄H₉-C≡C-CO-CH=CH-CH₃ ;
D : 7-tetradecyn-6-one de formule C₆H₁₃-C≡C-CO-C₅H₁₁

### III-2 Test d'efficacité sur la butylamine :

| | % de mauvaise odeur restante |
|---|---|
| 1 g de butylamine (Référence) | 100 |
| 1 g de butylamine + 1 g de A | 30 |
| 1 g de butylamine + 1 g de B | 28 |
| 1 g de butylamine + 1 g de C | 25 |
| 1 g de butylamine + 1 g de D | 58 |

On note une nette diminution de l'odeur résiduelle

### III-3 Test d'efficacité sur l'acide thioglycolique :

| | % de mauvaise odeur restante |
|---|---|
| 1 g d'acide thioglycolique (Référence) | 100 |
| 1 g d'acide thioglycolique + 1 g de A | 57 |
| 1 g d'acide thioglycolique + 1 g de B | 30 |
| 1 g d'acide thioglycolique + 1 g de C | 57 |
| 1 g d'acide thioglycolique + 1 g de D | 49 |

On note une nette diminution de l'odeur résiduelle

### III- Test d'efficacité sur une mauvaise odeur de cuisine :

Ce test vise à montrer l'effet potentialisateur des cétones acétyléniques sur un déodorant déjà connu, le neutral 1.

Une mauvaise odeur de cuisine est préparée en mélangeant les différents composés suivants :

| **Composé** | **% dans le mélange** | **Caractéristique odorante** |
|---|---|---|
| Trimethylamine | 0,10 | Poisson |
| Dimethyl sulphide | 0,13 | Chou |
| Dimethyl disulphide | 0,02 | Oignon |
| Pyridine, 2-methanethiol 10% | 0,50 | Viande pourrie |
| Isovaleric acid | 0,70 | Sueur, Fromage |
| Butyric acid | 0,30 | Fromage |
| Capric acid | 0,50 | Fromage, Fermenté |
| Methyl amyl cetone | 0,10 | Fromage bleu intense |
| 2-Mercaptopropanone | 0,06 | Viande, Soufré, Moisi |
| Diallyl disulphide | 0,05 | Ail |
| Butvl butyryllactate | 1,30 | Lait tourné |
| Methional | 0,02 | Vieille pomme de terre |
| Indole 1% | 0,14 | Végétal pourri, moisi |
| Diacetyte | 0,10 | Beurre rance |
| Hexanoic acid | 0,20 | Vieux fromage |
| Guaïacol | 0,04 | Viande cuite |
| Dipropylen glycol | 95,74 | qsp 100 |

20 µL de cette mauvaise odeur sont déposés sur un support de cellulose de 1 cm², lequel est mis dans une cabine d'olfaction de 3 m³. Cette enceinte sert de référence.

De la même façon, des enceintes identiques closes contenant de la mauvaise odeur à laquelle 10 µL de neutral 1 seul, soit 10 µL d'un mélange de neutral et d'une cétone acétylénique (en proportion 99/1) sont également préparées.

Le panel de testeurs évalue ensuite l'odeur dans chacune des enceintes, selon l'ordre défini par un carré latin et en comparaison avec l'enceinte de référence contenant la mauvaise odeur seule.

Les cétones acétyléniques suivantes sont testées sur la solution malodorante de référence :
E : Neutral 1
F : Neutral 1 et 2,2-dimethyl-4-hexyn-3-one de formule CH₃-C≡C-CO-C(CH₃)₃
G : Neutral 1 et 5-decyn-4-one de formule C₄H₉-C≡C-CO-C₃H₇
H : Neutral 1 et 2,5-heptadiyn-4-one de formule H₃C-C≡C-CO-C≡C-CH₃
I : Neutral 1 et 3-octyn-2-one de formule C₄H₉-C≡C-CO-CH₃

**Formule du neutral 1 :**

| **Composé** | **Quantité (vol./vol.)** |
|---|---|
| Phenylacetaldehyde (A) | 0,32% |
| Hydroxycitronellal (A) | 1,63% |
| α-Hexylcinnamaldehyde (B) | 19,09% |
| Lilial (A) | 7,91% |
| Dipropylen glycol | qsp 100 |

| | **% de mauvaise odeur restante** |
|---|---|
| Odeur de cuisine seule (OdC =Référence) | 100 |
| OdC + E | 39 |
| OdC + F | 32 |
| OdC + G | 24 |
| OdC + H | 30 |
| OdC + I | 36 |

L'ajout d'une petite quantité de cétone acétylénique en plus du neutral améliore l'efficacité du neutral 1 contre la mauvaise odeur de cuisine.

### III-5 Test d'efficacité sur une mauvaise odeur de tabac :

Une mauvaise odeur de tabac est préparée à partir de cendre et de mégots de cigarettes. 20 g de cette odeur sont déposés dans une cabine d'olfaction de 3 m³.

Des serviettes en tissu sont déposées dans l'enceinte et laissées 24 h afin qu'elle s'imprègne de l'odeur de tabac.

100 µL de Neutral 2 sont ensuite pulvérisés sur le linge.

De même des serviettes mises en contact avec l'odeur de tabac sont préparées et des mélanges de Neutral 2 anti-tabac et d'une petite quantité de cétone acétylénique (proportion 99/1) ou d'un mélange de deux cétones acétyléniques (proportion 99/0,5/0,5) sont pulvérisés sur les serviettes.

Le panel évalue alors la quantité de mauvaise odeur de tabac sur les serviettes en comparaison avec les serviettes n'ayant pas reçu de traitement contre la mauvaise odeur.

Les cétones acétyléniques suivantes sont testées sur la solution malodorante de référence :
J : Neutral 2,
K : Neutral 2 et 2,5-heptadiyn-4-one de formule H₃C-C≡C-CO-C≡C-CH₃,
L : Neutral 2 et 1-octyn-3-one de formule C₅H₁₁ -CO-C=CH,
M : Neutral 2 et 5-decyn-4-one de formule C₄H₉-C≡C-CO-C₃H₇,
N : Neutral 2 et 1-phenyl-2-propyn-1-one de formule C₆H₅-CO-C≡CH, et 6-phenyl-5-hexyn-2,4-dione de formule C₆H₅-C≡C-CO-CH₂-CO-CH₃,
O : Neutral 2 et 4-phenyl-3-butyne-2-one de formule C₆H₅-C≡C-CO-CH₃, et 1-phenyl-2-propyn-1-one de formule C₆H₅ -CO-C=CH,

**Formule du neutral 2 :**

| **Composé** | **Quantité (vol./vol.)** |
|---|---|
| Lilial (A) | 10% |
| Anisaldehyde (B) | 20% |
| α-Hexylcinnamaldehyde (B) | 69% |
| Butylhydroxytoluene | qsp 100 |

**Formule de pulvérisation :**

| **Composé** | **Quantité (v/v)** |
|---|---|
| Ethyl alcohol | 15,0 |
| Sodium lauroyl sarcosinate | 0,5 |
| Sodium carbonate | 2,0 |
| Sodium dioctyl sulfosuccinate | 0,5 |
| Neural 2 | 0,5 |
| Eau déminéralisée | qsp100 |

| | % de mauvaise odeur restante |
|---|---|
| Odeur de Tabac seule (OdT =Référence) | 100 |
| OdT + J | 65 |
| OdT + K | 33 |
| OdT + L | 39 |
| OdT + M | 51 |
| OdT + N | 51 |
| OdT + O | 55 |

L'efficacité du Neutral 2 est améliorée par ajout d'au moins une cétone acétylénique.

### III-6 Test d'efficacité sur une mauvaise odeur de toilettes :

Une mauvaise odeur de toilettes est préparée en mélangeant les différents composés suivants :

| **Composé** | **% dans le mélange** | **Charactèristique odorante** |
|---|---|---|
| Scatole | 0,90 | Fécale |
| Thionaphtol | 0,90 | Fécale, soufré |
| Thioglycolic acid | 21,18 | Soufré |
| Hexanoic acid | 6,00 | Sueur |
| p-Cresyl isovalerate | 2,18 | Fécale, sueur |
| N-Methyl morpholine | 6,00 | Urine |
| Dipropylen glycol | qsp 100 | |

20 µL de cette mauvaise odeur sont déposés dans l'eau d'une cuvette de toilette en cabine. Dans une autre cabine, 20 µL de mauvaise odeur sont déposés ainsi que 20 µL d'un neutral 3 actif contre la mauvaise odeur de toilettes.

Dans d'autres cabines, on ajoute en plus une cétone acétylénique en proportion (99/1) au neutral 3.

La quantité de mauvaise odeur de toilettes dans chaque cabine est ensuite évaluée par le panel de testeurs.

Les cétones acétyléniques suivantes sont testées sur la solution malodorante de référence :
P : neutral 3 ;
Q : neutral 3 et 2,5-heptadiyn-4-one de formule H₃C-C≡C-CO-C≡C-CH₃ ;
R : neutral 3 et 2,2-dimethyl-4-hexyn-3-one de formule CH₃-C≡C-CO-C(CH₃)₃ ;
S : neutral 3 et 1-octyn-3-one de formule C₅H₁₁-CO-C≡CH ;
T : neutral 3 et 5-decyn-4-one de formule C₄H₉-C≡C-CO-C₃H₇ ;

**Formule du neutral 3 :**

| **Composé** | **Quantité (vol./vol.)** |
|---|---|
| Citral (B) | 3,5% |
| Orange terpenes | 1,5% |
| Linalyl acetate | 11,0% |
| Linalol | 23,0% |
| Citral DEA | 0,3% |
| Eugenol | 5,0% |
| 3Z-Hexenyl acetate 10% DPG | 5,0% |
| α-Hexyl cinnamaldehyde (B) | 15,0% |
| Hederile 10% DPG | 1,0% |
| Butylhydroxytoluene 10% DPG | 0,2% |
| Dipropylen glycol | qsp 100 |

| | % de mauvaise odeur restante |
|---|---|
| Odeur de toilette seule (OdT =Référencel | 100 |
| (OdT + P | 47 |
| OdT + Q | 19 |
| OdT + R | 20 |
| OdT + S | 21 |
| OdT + T | 23 |

L'efficacité du neutral 3 est améliorée par ajout d'au moins une cétone acétylénique.

### IV- Réactivité chimique

Des essais parallèles contribuent à relater la réactivité des cétones acétyléniques. Ils sont réalisés en faisant réagir mole à mole une cétone acétylénique avec une substance responsable d'une mauvaise odeur. La réaction est réalisée à température ambiante dans le THF.

40 cétones acétyléniques sont testées en vue de vérifier leur réactivité (leur potentialité à diminuer les mauvaises odeurs).

### IV-1 Réactivité avec la piperidine

Un substrat, la pipéridine, est utilisé pour vérifier cette réactivité. La réactivité est mesurée par le signal obtenu en chromatographie en phase gazeuse (signal respectif obtenu après un temps de réaction t = 5 min, t = 2 h et t = 5 h mesuré pour la cétone acétylénique, pour le produit de réaction).

### IV-1.1 Temps de réaction 5 minutes

| **Composés** | **t = 5 min** |
|---|---|
| C₆H₅-CO-C≡CH | 0 |
| CH₃-C≡C-CO-C≡C-CH₃ | 0 |
| CH₃O-C₆H₄-CO-C≡CH | 0 |
| C₆H₅-C≡C-CO-C₄H₃O (furyl) | 0 |
| C₃H₇-C≡C-CO-C≡C-C₃H₇ | 0 |
| C₆H₅-CO-C≡C-CO-C₆H₅ | 1 |
| C₇H₁₅-CO-C≡CH | 4 |
| C₅H₁₁-C≡C-CO-C₄H₃₀ (furyl) | 5 |
| C₅H₁₁-CO-C≡CH | 8 |
| CH₃-C≡C-CO-C₆H₄-(p-CH₃) | 9 |

Cinq cétones acétyléniques montrent une très forte réactivité vis-à-vis du substrat utilisé. 5 min après avoir mis en contact les deux réactifs, la disparition de la cétone acétylénique est totale et la formation de la substance de réaction atteint son maximum.

Cinq autres cétones acétyléniques montrent une forte réactivité avec une disparition de 90 à 99% des réactifs après 5 min.

### IV-1.2 Temps de réaction 2 heures

L'examen de la réactivité après 2 heures de réaction montre la disparition de plus de 90 % des cétones acétyléniques (et l'apparition du produit de réaction) avec plus de la moitié des produits testés (24 sur 40).

| | |
|---|---|
| **Composés** | **t = 2 h** |
| C₆H₅-CO-C≡CH | 0 |
| CH₃-C≡C-CO-C≡C-CH₃ | 0 |
| CH₃O-C₆H₄-CO-C≡CH | 0 |
| C₅H₁₁-C≡C-CO-C₄H₃O (furyl) | 0 |
| CH₃-C≡C-CO-C₆H₃(OCH₃)₂ | 0 |
| C₆H₅-CO-C≡C-CO-C₆H₅ | 0 |
| C₃H₅-C≡C-CO-C₆H₄-CH₃ | 0 |
| C₆H₅-C≡C-CO-C₄H₃O (furyl) | 0 |
| CH₃-C≡C-CO-C₆H₄-(p-CH₃) | 0 |
| C₃H₇-C≡C-CO-C≡C-C₃H₇ | 0 |
| C₂H₅-CO-C≡C-(CH₂)₄-C≡C-CO-C₂H₅ | 1 |
| C₆H₅-C≡C-CO-C₂H₅ | 1 |
| C₆H₅-C≡C-CO-CH₂-CO-CH₃ | 2 |
| C₆H₅-C≡C-CO-CH₃ | 2 |
| CH₃-C≡C-CO-C(CH₃)₃ | 2 |
| CH₃-C≡C-CO-C₅H₁₁ | 4 |
| C₇H₁₅-CO-C≡CH | 4 |
| C₃H₇-CH₂-C≡C-CO-CH(CH₃)₂ | 5 |
| C₃H₇-CO-C≡C-CH₂-CH(CH₃)₂ | |
| C₅H₁₁-CO-C≡CH | 7 |
| C₆H₅-C≡C-CO-C₄H₉ | 7 |
| C₃H₅-C≡C-CO-CH(C₂H₅)-C₄H₉ | 8 |
| (CH₃)₂CH-C₂H₄-C≡C-CO-CH₂-CH(CH₃)₂ | 8 |
| C₅H₁₁-C≡C-CO-C₂H₅ | 9 |
| C₆H₁₃-C≡C-CO-C₇H₁₅ | 9 |

### IV-1.3 Temps de réaction 5 heures

L'examen de la réactivité après 5 heures de réaction montre la disparition de plus de 90 % des cétones acétyléniques (et l'apparition du produit de réaction) avec la quasi-totalité des produits testés (36 sur 40).

Trois cétones acétyléniques réagissent fortement avec une disparition de 55 à 83%.

Une seule substance réagit faiblement.

| **Composés** | **t = 5 h** |
|---|---|
| CH₃-C≡C-CO-C(CH₃)₃ | 0 |
| CH₃-C≡C-CO-C≡C-CH₃ | 0 |
| CH₃O-C₆H₄-CO-C≡CH | 0 |
| C₅H₁₁-C≡C-CO-C₄H₃O (furyl) | 0 |
| CH₃-C≡C-CO-C₆H₃(OCH₃)₂ | 0 |
| C₆H₅-CO-C≡C-CO-C₆Hₛ | 0 |
| C₃H₅-C≡C-CO-C₆H₄-(p-CH₃) | 0 |
| C₆H₅-C≡C-CO-C₄H₃O (furyl) | 0 |
| CH₃-C≡C-CO-C₆H₄-(p-CH₃) | 0 |
| C₃H₇-C≡C-CO-C≡C-C₃H₇ | 0 |
| C₂H₅-CO-C≡C-(CH₂)₄-C≡C-CO-C₂H₅ | 0 |
| C₆H₅-CO-C≡CH | 0 |
| C₆H₅-C≡C-CO-CH₃ | 0 |
| C₅H₁₁-C≡C-CO-C₂H₅ | 1 |
| C₃H₅-C≡C-CO-CH(C₂H₅)-C₄H₉ | 1 |
| C₆H₁₃-C≡C-CO-C₇H₁₅ | 1 |
| CH₃-C≡C-CO-C₅H₁₁ | 1 |
| C₆H₅-C≡C-CO-CH₂-CO-CH₃ | 1 |
| C₆H₅-C≡C-CO-C₂H₅ | 1 |
| C₆H₁₃-C≡C-CO-C₅H₁₁ | 2 |
| C₄H₉-C≡C-CO-CH₃ | 2 |
| C₂H₅-CO-C≡C₆(CH₂)₄-C≡CH | 2 |
| C₄H₉-C≡C-CO-(CH₂)₂-C₅H₉ | 3 |
| C₃H₇-C≡C-CO-C₄H₉ | 3 |
| C₃H₇-CH₂-C≡C-CO-CH(CH₃)₂ | 4 |
| C₃H₇-CO-C≡C-CH₂-CH(CH₃)₂ | |
| C₇H₁₅-CO-C≡CH | 4 |
| C₃H₇-C≡C-CO-CH(CH₃)₂ | 6 |
| C₄H₉-C≡C-CO-C₃H₇ | 6 |
| C₄H₉-C≡C-CO-C₃H₅ (cyclopropyl) | 7 |
| (CH₃)₂CH-C₂H₄-C≡C-CO-CH₂-CH(CH₃)₂ | 7 |
| C₆H₅-C≡C-CO-C₄H₉ | 7 |
| C₅H₁₁-CO-C≡CH | 8 |
| C₄H₉-C≡C-CO-C₆H₁₁ | 8 |
| (CH₃)₂CH-C₂H4-C≡C-CO-C(CH₃)₃ | 9 |
| C₃H₇-CH₂-C≡C-CO-C₂H₅ | 10 |
| C₃H₇-CO-C≡C-CH₂-C₂H₅ | |
| C₄H₉-C≡C-CO-CH≡CH-CH₃ | 17 |
| (CH₃)₂CH-C₂H₄-C≡C-CO-CH(C₂H₅)-C₄H₉ | 23 |
| C₆H₁₁-C≡C-CO-CH(C₂H₅)₂ | 45 |
| (CH₃)₂CH-CO-C≡C-CH₂-O-CH₂-C≡C-CO-CH(CH₃)₂ | 87 |

### IV-2 Réactivité avec les mercaptans

Trois composés soufrés servent de support pour la réactivité des cétones acétyléniques (butanethiol, 2-phenylethanethiol et α-toluenethiol).

Cinq cétones acétyléniques qui réagissaient fortement avec la pipéridine ont été testées.

Les conditions opératoires sont identiques à celles précédemment décrites pour la pipéridine (Réaction mole à mole d'une cétone acétylénique avec un mercaptan).

La réaction est réalisée à température ambiante dans le THF).

Le suivi de la réactivité est réalisé par mesures chromatographiques qui sont réalisées pendant 3 heures après les temps de réaction suivants :
t = 5min, t = 40 min, t = 75 min, t = 110 min, t = 145min, t = 180min.

### IV-2.1. Réactivité avec le 2-phényléthanethiol

| | **Concentration des cétones acétyléniques (% GC)** | | | | | |
|---|---|---|---|---|---|---|
| **Temps (min)** | **5** | **40** | **75** | **110** | **145** | **180** |
| C₆H₅-CO-C≡CH | 3 | - | - | - | - | - |
| CH₃O-C₆H₄-CO-C≡CH | 6 | - | - | - | - | - |
| CH₃-C≡C-CO-C≡C-CH₃ | 90 | 20 | 2 | - | - | - |
| C₆H₅-C≡C-CO-C₄H₃O (furyl) | 88 | 35 | 24 | 13 | - | - |
| C₃H₇-C≡C-CO-C≡C-C₃H₇ | 87 | 46 | 40 | 35 | 29 | 26 |

Deux de ces substances réagissent instantanément, deux autres disparaissent après 2 heures de réaction, la cinquième réagit fortement seulement après 3 heures de réaction.

Trois autres cétones acétyléniques, 1-cyclopentyl-4-nony-3-one, (C₄H₉-C≡C-CO-C₂H₄-C₅H₉), 1-cyclopropyl-2-heptyn-1-one (C₄H₉-C≡C-CO-C₃H₅) et 5-decyn-4-one (C₄H₉-C≡C-CO-C₃H₇) ne réagissent pas avec le 2-phenylethanethiol. L'addition d'un catalyseur favorise très légèrement leur réactivité.

### IV-2.2. Réactivité avec le butanethiol

| | **Concentration des** cétones acétyléniques **(% GC)** | | | | | |
|---|---|---|---|---|---|---|
| **Temps (mn)** | **5** | **40** | **75** | **110** | **145** | **180** |
| C₆H₅-CO-C≡CH | 2 | - | - | - | - | - |
| CH₃O-C₆H₄-CO-C≡CH | 12 | tr | - | - | - | - |
| CH₃-C≡C-CO-C≡C-CH₃ | 78 | 14 | 6 | 2 | - | - |
| C₆H₅-C≡C-CO-C₄H₃O (furyl) | 95 | 82 | 67 | 53 | 45 | 39 |
| C₃H₇-C≡C-CO-C≡C-C₃H₇ | 96 | 86 | 75 | 60 | 44 | 40 |

La réactivité avec le butanethiol est assez similaire à celle du 2-phenylethanethiol.

### IV-2.3. Réactivité avec le toluenethiol

| | **Concentration des cétones acétyléniques (% GC)** | | | | | |
|---|---|---|---|---|---|---|
| **Temps (mn)** | **5** | **40** | **75** | **110** | **145** | **180** |
| CH₃-C≡C-CO-C≡C-CH₃ | 2 | - | - | - | - | - |
| C₆H₅-C≡C-CO-C₄H₃O (furyl) | 16 | - | - | - | - | - |

La réaction avec le toluenethiol est très rapide avec deux cibles, plus lente avec les trois autres cibles testées.

### V- Tests chromatographiques

Chromatographie en phase gaz : utilisation d'un étalon interne, le décanoate d'éthyle.

Les pics correspondant à chaque cétone acétylénique, à la butylamine, à l'étalon interne et au solvant sont déterminés.

Les surfaces des pics permettent de déterminer la quantité de butylamine (mauvaise odeur) résiduelle.

Les analyses sont faites chromatographies toutes les 25 min. afin d'établir des cinétiques.

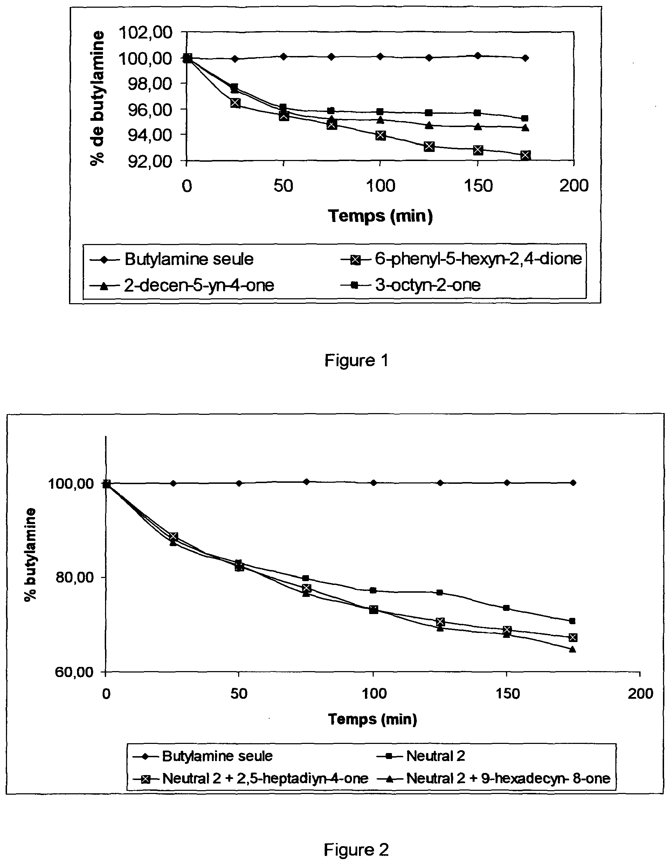

Les résultats sont présentés à la figure 1.

Utilisées seules en proportion 1/10 (cétone acétylénique / Butylamine), les cétones acétyléniques entraînent une baisse de 4 à 8% de la quantité de butylamine en moins de 3h.

L'effet du neutral 2 seul sur la butylamine et l'effet du neutral 2 auquel est ajouté 1 % d'une cétone acétylénique sont testés en comparaison des essais précédents.

Les résultats sont présentés à la figure 2.

On constate une augmentation de l'efficacité du neutral 2 sur la quantité de butylamine.

Les essais décrits aux paragraphes IV et V démontrent que les cétones acétyléniques exerce leur pouvoir désodorisant en détruisant physiquement les molécules malodorantes. Cet effet n'est donc pas un simple effet masquant.

### VI- Compositions désodorisante

### VI-1. Aérosol désodorisant d'atmosphère

On a réalisé un spray d'ambiance aqueux parfumé avec un neutralisant "magnolia" contenant les aldéhydes suivants :

| | |
|---|---|
| Phenylacetaldehyde (A) | 0,32% |
| Hydroxycitronellal (A) | 1,63% |
| α-Hexylcinnamaldehyde (B) | 19,09% |
| Lilial (A) | 7,91% |
| 2,4-Dimethyl-3-cyclohexen-1-carboxaldehyde (A) | 0,15% |
| Dipropylen glycol | qsp 100 |

### VI-2. Désodorisant et désinfectant liquide pour sols :

Les aldéhydes peuvent également être formulés sous la forme d'acétals afin de réduire l'odeur intrinsèque du neutralisant et d'augmenter la durée d'action. Il est connu qu'un acétal s'hydrolyse en milieu acide en libérant l'aldéhyde correspondant.

Voici à titre d'exemples quelques acétals faiblement odorants pouvant être utilisés dans un désodorisant de surface : propionaldehyde DEA, phenylacetaldehyde-méthanol acétal, citral diéthylacetal, hydroxycitronnellal diméthylacetal, benzatdehyde diéthylacetal.

**Formule de désodorisant de surface :**

| **Composé** | **Quantité (v/v)** |
|---|---|
| Ethyl alcohol | 15,0 |
| Sodium lauroyl sarcosinate | 0,5 |
| Sodium carbonate | 2,0 |
| Sodium dioctyl sulfosuccinate | 0,5 |
| Neutral 2 | 0,5 |
| Eau déminéralisée | qsp100 |

## Revendications

1. Utilisation, dans une composition désodorisante, pour détruire les molécules malodorantes d'au moins un composé de la famille des cétones α-acétyléniques comprenant au moins un enchainement -CO-C≡C- ou -C=C-CO-, répondant à la formule générale 1
**R-(CO)ₖ-C**≡**C-(A)-(C**≡**C)ₘ-(CO)ₙ-R₁** **(1)**
dans laquelle
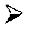 R et R₁ peuvent représenter, indépendamment ou simultanément, un radical choisi parmi
o une chaîne alkyle comprenant de 1 à 9, préférentiellement de 3 à 7 atomes de carbone, linéaire ou ramifiée, substituée ou non, ou
o un cycloalcane comprenant de 3 à 8, préférentiellement de 5 ou 6 atomes de carbone, substitué ou non ; ou
o un cycle furanique, saturé ou non, substitué ou non ; ou
o un cycle pyranique, saturé ou non, substitué ou non ; ou
o un cycle aromatique comprenant de 6 à 8 atomes de carbone, substitué ou non ; ou encore
o un atome d'hydrogène.
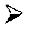 A représente un groupement choisi parmi -(CH₂)ₓ avec x représentant un entier de valeur 0 à 6, préférentiellement de 0 à 4, (-CO)l avec l représentant un entier de valeur 0 ou 1, ou encore un enchainement -(CH₂)_{y}-O-(CH₂)_{z}-avec y et z représentant, simultanément ou indépendamment, un entier de valeur 0 à 6, préférentiellement de 0 à 4, étant entendu que x + y ne dépasse pas la valeur 6 ;
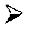 k, m et n sont, simultanément ou indépendamment, un entier de valeur 0 ou 1, étant entendu que k et n ne peuvent être simultanément de valeur 0 si l est égal à 0 ;
à l'exception de la 2-méthyl-4-nonyn-3-one de formule C₄H₉-C≡C-CO-CH(CH₃)₂.

2. Utilisation selon la revendication 1, **caractérisée en ce que** le composé de formule 1 est choisi parmi
- 1-octyn-3-one de formule C₅H₁₁-CO-C=CH ;
- 3-octyn-2-one de formule C₄H₉-C≡C-CO-CH₃ ;
- 4-octyn 3-one de formule C₃H₇-C≡C-CO-C₂H₅ ;
- 4-nonyn-3-one de formule C₃H₇-CH₂-C≡C-CO-C₂H₅ ;
- 2-nonyn-4-one de formule CH₃-C≡C-CO-C₅H₁₁;
- 5-nonyn-4-one de formule C₃H₇-CO-C≡C-CH₂-C₂H₅ ;
- 1-decyn-3-one de formule C₇H₁₅-CO-C≡CH ;
- 4-decyn-3-one de formule C₅H₁₁-C≡C-CO-C₂H₅ ;
- 6-decyn-5-one de formule C₃H₇-C≡C-CO-C₄H₉ ;
- 5-decyn-4-one de formule C₄H₉-C≡C-CO-C₃H₇ ;
- 1-(p-methoxyphenyl)-2-propyn-1-one de formule H₃C-O-C₆H₄-CO-C≡CH ;
- 1-cyclopropyl-2-heptyn-1-one de formule C₄H₉-C≡C-CO-C₃H₅ cyclopropyl) ;
- 2-methyl-5-decyn-4-one de formule C₄H₉-C≡C-CO-CH₂-CH(CH₃)₂ ;
- 9-methyl-5-decyn-4-one de formule C₃H₇-CO-C≡C-CH₂-CH₂-CH(CH₃)₂ ;
- 2-methyl-4-octyn-3-one de formule C₃H₇-C≡C-CO-CH(CH₃)₂ ;
- 1-phenyl-2-propyn-1-one de formule C₆H₅ -CO-C=CH ;
- 4-phenyl-3-butyn-2-one de formule C₆H₅-C≡C-CO-CH₃ ;
- 5-phenyl-4-pentyn-3-one de formule C₆H₅-C≡C-CO-C₂H₅ ;
- 1-phenyl-1-heptyn-3-one de formule C₆H₅-C≡C-CO-C₄H₉ ;
- 1-(2-furyl)-3-phenyl-2-propyn-1-one de formule C₆H₅-C≡C-CO-C₄H₃O furyl) ;
- 1-(2-furyl)-2-octyn-1-one de formule C₅H₁₁-C≡C-CO-C₄H₃O (furyl) ;
- C₆H₅-C≡C-CO-C₄H₃O (furyl) ;
- 3-cyclopropyl-1-(p-tolyl)-2-propyn-1-one de formule C₃H₂-C≡C-CO-C₆H₄-CH₃ ;
- 1-cyclopropyl-4-methyl-1-hexyn-3-one de formule C₃H₅-C≡C-CO-CH(C₂H₅)-CH₃ (cyclopropyl) ;
- 9-hexadecyn-8-one de formule C₆H₁₃-C≡C-CO-C₇H₁₅ ;
- 5-ethyl-11-methyl-7-dodecyn-6-one de formule (CH₃)₂CH-C₂H₄-C≡C-CO-CH(C₂H₅)-C₄H₉;
- 7-tetradecyn-6-one de formule C₆H₁₃-C=C-CO-C₅H₁₁ ;
- 1-cyclohexyl-4-ethyl-1-hexyn-3-one de formule C₆H₁₁-C=C-CO-CH(C₂H₅)₂ (cyclohexyl) ;
- 1-cyclopentyl-4-nonyn-3-one de formule C₄H₉-C≡C-CO-C₂H₄-C₅H₉ (cyclopentyl) ;
- 1-cyclohexyl-2-heptyn-1-one de formule C₄H₉-C≡C-CO-C₆Hₙ (cyclohexyl) ;
- 1-(p-tolyl)-2-butyn-1-one de formule CH₃-C≡C-CO-C₆H₄-(p-CH₃);
- 2,2,8-trimethyl-4-nonyn-3-one de formule (CH₃)₂CH-C₂H₄-C≡C-CO-C(CH₃)₃ ;
- 2,2-dimethyl-4-hexyn-3-one de formule CH₃-C≡C-CO-C(CH₃)₃ ;
- 1,4-diphenyl-2-butyn-1,4-dione de formule C₆H₅-CO-C≡C-CO-C₆H₅ ;
- 5-decyn-2,4-dione de formule C₄H₉-C≡C-CO-CH₂-CO-CH₃ ;
- 1-(3,4-dimethoxyphenyl)-2-butyn-1-one de formule CH₃-C≡C-CO-C₆H₃-(OCH₃)₂ ;
- 2,5-heptadiyn-4-one de formule H₃C-C≡C-CO-C≡C-CH₃ ;
- 3-hexyn-2,5-dione de formule CH₃-CO-C≡C-CO-CH₃ ;
- 4-octyn-3,6-dione de formule C₂H₅-CO-C≡C-CO-C₂H₅ ;
- 2,7-dimethyl-4-octyn-3,6-dione de formule (CH₃)₂CH-CO-C≡C-CO-CH(CH₃)₂ ;
- 2,7-diméthyl-4-octyn-3-one de formule (CH₃)₂CH-CH₂-C≡C-CO-CH(CH₃)₂ ;
- 4,7-undecadiyn-6-one de formule C₃H₇-C≡C-CO-C≡C-C₃H₇ ;
- 5-decyn-4,7-dione de formule C₃H₇-CO-C≡C-CO-C₃H₇ ;
- 2,9-dimethyl-5-decyn-4,7-dione de formule (CH3)₂CH-CH₂-CO-C≡C-CO-CH₂-CH(CH₃)₂ ;
- 6-dodecyn-5,8-dione de formule C₄H₉-CO-C≡C-CO-C₄H₉ ;
- 2,11-dimethyl-6-dodecyn-5,8-dione de formule (CH₃)₂CH-C₂H₄-CO-C≡C-CO-(CH₂)₂-CH(CH₃) ;
- 2,9-dimethyl-5-decyn-4-one de formule (CH₃)₂CH-C₂H₄-C≡C-CO-CH₂-CH(CH₃)₂ ;
- 7-tetradecyn-6,9-dione de formule C₅H₁₁-CO-C≡C-CO-C₅H₁₁ ;
- 4,10-undecadiyn-3-one de formule HC≡C-C₄H₈-C≡C-CO-C₂H₅ ;
- 2,12-dimethyl-7-oxa-4,9-tridecadiyn-3,11-dione de formule (CH₃)₂-CO-C≡C-CH₂-O-CH₂-C≡C-CO-CH(CH₃)₂, ou encore
- 4,10-tetradecadiyn-3,12-dione de formule C₂H₅-CO-C≡C-C₄H₈-C≡C-CO-C₂H₅.

3. Utilisation selon l'une quelconque des revendications 1 ou 2, **caractérisée en ce que** le composé de formule 1 est un couple de composé de formule 1 choisi parmi les couples
- 1-phenyl-2-propyn-1-one / 1-decyn-3-one ;
- 1-(2-furyl)-2-octyn-1-one / 1-decyn-3-one ;
- 1-(2-furyl)-3-phenyl-2-propyn-1-one / 1-decyn-3-one ;
- 1-octyn-3-one /1-decyn-3-one ;
- 1-(p-tolyl)-2-butyn-1-one /1-decyn-3-one ;
- 2,5-heptadiyn-4-one /1-decyn-3-one ;
- 1-(p-methoxyphenyl)-2-propyn-1-one / 1-decyn-3-one ;
- 1,4-diphenyl-2-butyn-1,4-dione / 1-decyn-3-one ;
- 4,7-undecadiyn-6-one / 1-decyn-3-one ;
- 1-(2-furyl)-2-octyn-1-one / 1-phenyl-2-propyn-1-one ;
- 1-(2-furyl)-3-phenyl-2-propyn-1-one / 1-phenyl-2-propyn-1-one ;
- 1-octyn-3-one / 1-phenyl-2-propyn-1-one ;
- 1-(p-tolyl)-2-butyn-1-one / 1 -phenyl-2-propyn-1-one ;
- 2,5-heptadiyn-4-one / 1-phenyl-2-propyn-1-one ;
- 1-(p-methoxyphenyl)-2-propyn-1-one / 1-phenyl-2-propyn-1-one ;
- 1,4-diphenyl-2-butyn-1,4-dione / 1-phenyl-2-propyn-1-one ;
- 4,7-undecadiyn-6-one / 1-phenyl-2-propyn-1-one ;
- 1-(2-furyl)-3-phenyl-2-propyn-1-one / 1-(2-furyl)-2-octyn-1-one ;
- 1-octyn-3-one/ 1-(2-furl)-2-octyn-1-one 1-(p-tolyl)-2-butyn-1-one ;
- 2,5-heptadiyn-4-one / 1-(2-furyl)-2-octyn-1-one ;
- 1-(p-methoxyphenyl)-2-propyn-1-one / 1-(2-furyl)-2-octyn-1-one
- 1,4-diphenyl-2-butyn-1,4-dione / 1-(2-furyl)-2-octyn-1-one ;
- 4,7-undecadiyn-6-one / 1-(2-furyl)-2-octyn-1-one ;
- 1-octyn-3-one / 1-(2-furyl)-3-phenyl-2-propyn-1-one ;
- 1-(p-tolyl)-2-butyn-1-one / 1-(2-furyl)-3-phenyl-2-propyn-1-one ;
- 2,5-heptadiyn-4-one / 1-(2-furyl)-3-phenyl-2-propyn-1-one ;
- 1-(p-methoxyphenyl)-2-propyn-1-one / 1-(2-furyl)-3-phenyl-2-propyn-1-one ;
- 1,4-diphenyl-2-butyn-1,4-dione / 1-(2-furyl)-3-phenyl-2-propyn-1-one ;
- 4,7-undecadiyn-6-one / 1-(2-furyl)-3-phenyl-2-propyn-1-one ;
- 1-(p-tolyl)-2-butyn-1-one / 1-octyn-3-one ;
- 2,5-heptadiyn-4-one / 1-octyn-3-one ;
- 1-(p-methoxyphenyl)-2-propyn-1-one / 1-octyn-3-one ;
- 1,4-diphenyl-2-butyn-1,4-dione / 1-octyn-3-one ;
- 4,7-undecadiyn-6-one / 1-octyn-3-one ;
- 2,5-heptadiyn-4-one / 1-(p-tolyl)-2-butyn-1-one ;
- 1-(p-methoxyphenyl)-2-propyn-1-one / 1-(p-tolyl)-2-butyn-1-one ;
- 1,4-diphenyl-2-butyn-1,4-dione / 1-(p-tolyl)-2-butyn-1-one ;
- 4,7-undecadiyn-6-one / 1-(p-tolyl)-2-butyn-1-one ;
- 1-(p-methoxyphenyl)-2-propyn-1-one / 2,5-heptadiyn-4-one ;
- 1,4-diphenyl-2-butyn-1,4-dione / 2,5-heptadiyn-4-one ;
- 4,7-undecadiyn-6-one / 2,5-heptadiyn-4-one ;
- 1,4-diphenyl-2-butyn-1,4-dione / 1-(p-methoxyphenyl)-2-propyn-1-one ;
- 4,7-undecadiyn-6-one / 1-(p-methoxyphenyl)-2-propyn-1-one ;
- 4,7-undecadiyn-6-one / 1,4-diphenyl-2-butyn-1,4-dione.

4. Utilisation selon l'une quelconque des revendications 1 à 3, **caractérisée en ce que** le composé de formule 1 est dans la composition en une quantité comprise entre 0,1% et 40%, préférentiellement entre 0,1% et 10%, en poids total de la composition.

5. Utilisation selon l'une quelconque des revendications 1 à 4, **caractérisée en ce que** la composition comprend en outre au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique, et encore plus préférentiellement dans une composition désodorisante comprenant un mélange d'au moins un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

6. Utilisation selon la revendication 5, **caractérisée en ce qu'**elle comprend une quantité de l'aldéhyde comprise entre 0,5% et 50% de la composition, préférentiellement en une quantité comprise entre 1% et 20% de la composition.

7. Utilisation selon l'une quelconque des revendications 5 ou 6, **caractérisée en ce que** les aldéhydes des classes A et B sont en proportions relatives l'un par rapport à l'autre en proportions de 80/20 à 20/80 notamment en proportions de 50/50 et dans une quantité totale d'aldéhydes comprise entre 1 % et 90 % de la composition, préférentiellement en une quantité comprise entre 10% et 30% de la composition.

8. Composition désodorisante **caractérisée en ce qu'**elle comprend au moins un composé de la famille des cétones α-acétyléniques répondant à la formule générale 1
**R-(CO)ₖ-C≡C-(A)-(C≡C)ₘ-(CO)ₙ-R₁** **(1)**
choisi parmi
- 1-octyn-3-one de formule C₅H₁₁-CO-C≡CH ;
- 3-octyn-2-one de formule C₄H₉-C≡C-CO-CH₃ ;
- 4-octyn 3-one de formule C₃H₇-C≡C-CO-C₂H₅ ;
- 4-nonyn-3-one de formule C₃H₇-CH₂-C≡C-CO-C₂H₅ ;
- 2-nonyn-4-one de formule CH₃-C≡C-CO-C₅H₁₁ ;
- 5-nonyn-4-one de formule C₃H₇-CO-C≡C-CH₂-C₂H₅ ;
- 1-decyn-3-one de formule C₇H₁₅-CO-C≡CH ;
- 4-decyn-3-one de formule C₅H₁₁-C≡C-CO-C₂H₅ ;
- 6-decyn-5-one de formule C₃H₇-C≡C-CO-C₄H₉ ;
- 5-decyn-4-one de formule C₄H₉-C≡C-CO-C₃H₇ ;
- 1-cyclopropyl-2-heptyn-1-one de formule C₄H₉-C≡C-CO-C₃H₅ cyclopropyl) ;
- 2-methyl-5-decyn-4-one de formule C₄H₉-C≡C-CO-CH₂-CH(CH₃)₂ ;
- 9-methyl-5-decyn-4-one de formule C₃H₇-CO-C≡C-CH₂-CH₂-CH(CH₃)₂ ;
- 2-methyl-4-octyn-3-one de formule C₃H₇-C≡C-CO-CH(CH₃)₂ ;
- 4-phenyl-3-butyn-2-one de formule C₆H₅-C≡C-CO-CH₃ ;
- 5-phenyl-4-pentyn-3-one de formule C₆H₅-C≡C-CO-C₂H₅ ;
- 1-phenyl-1-heptyn-3-one de formule C₆H₅-C≡C-CO-C₄H₉ ;
- 1-cyclopropyl-4-methyl-1-hexyn-3-one de formule C₃H₅-C≡C-CO-CH(C₂H₅)-CH₃ (cyclopropyl) ;
- 9-hexadecyn-8-one de formule C₆H₁₃-C≡C-CO-C₇H₁₅ ;
- 5-ethyl-11-methyl-7-dodecyn-6-one de formule (CH₃)₂CH-C₂H₄-C≡C-CO-CH(C₂H₅)-C₄H₉ ;
- 7-tetradecyn-6-one de formule C₆H₁₃-C≡C-CO-C₅H₁₁ ;
- 1-cyclohexyl-4-ethyl-1-hexyn-3-one de formule C₆H₁₁-C≡C-CO-CH(C₂H₅)₂ (cyclohexyl) ;
- 1-cyclopentyl-4-nonyn-3-one de formule C₄H₉-C≡C-CO-C₂H₄-C₅H₉ (cyclopentyl) ;
- 1-cyclohexyl-2-heptyn-1-one de formule C₄H₉-C≡C-CO-C₆H₁₁ (cyclohexyl) ;
- 2,2,8-trimethyl-4-nonyn-3-one de formule (CH₃)₂CH-C₂H₄-C≡C-CO-C(CH₃)₃ ;
- 2,2-dimethyl-4-hexyn-3-one de formule CH₃-C≡C-CO-C(CH₃)₃ ;
- 3-hexyn-2,5-dione de formule CH₃-CO-C≡C-CO-CH₃ ;
- 4-octyn-3,6-dione de formule C₂H₅-CO-C≡C-CO-C₂H₅ ;
- 2,7-dimethyl-4-octyn-3,6-dione de formule (CH₃)₂CH-CO-C≡C-CO-CH(CH₃)₂ ;
- 2,7-diméthyl-4-octyn-3-one de formule (CH₃)₂CH-CH₂-C≡C-CO-CH(CH₃)₂ ;
- 4,7-undecadiyn-6-one de formule C₃H₇-C≡C-CO-C≡C-C₃H₇ ;
- 5-decyn-4,7-dione de formule C₃H₇-CO-C≡C-CO-C₃H₇ ;
- 2,9-dimethyl-5-decyn-4,7-dione de formule (CH3)₂CH-CH₂-CO-C≡C-CO-CH₂-CH(CH₃)₂ ;
- 2,11-dimethyl-6-dodecyn-5,8-dione de formule (CH₃)₂CH-C₂H₄-CO-C≡C-CO-(CH₂)₂-CH(CH₃) ;
- 2,9-dimethyl-5-decyn-4-one de formule (CH₃)₂CH-C₂H₄-C≡C-CO-CH₂-CH(CH₃)₂ ;
- 7-tetradecyn-6,9-dione de formule C₅H₁₁-CO-C≡C-CO-C₅H₁₁ ;
- 4,10-undecadiyn-3-one de formule HC≡C-C₄H₈-C≡C-CO-C₂H₅ ;
- 2,12-dimethyl-7-oxa-4,9-tridecadiyn-3,11-dione de formule (CH₃)₂-CO-C≡C-CH₂-O-CH₂-C≡C-CO-CH(CH₃)₂, ou encore
- 4,10-tetradecadiyn-3,12-dione de formule C₂H₅-CO-C≡C-C₄H₈-C≡C-CO-C₂H₅.

9. Composition désodorisante **caractérisée en ce qu'**elle comprend au moins un composé de formule 1
**R-(CO)ₖ-C≡C-(A)-(C≡C)ₘ-(CO)ₙ-R₁** **(1)**
choisi parmi les couples
- 1-phenyl-2-propyn-1-one / 1-decyn-3-one ;
- 1-(2-furyl)-2-octyn-1-one / 1-decyn-3-one ;
- 1-(2-furyl)-3-phenyl-2-propyn-1-one / 1-decyn-3-one ;
- 1-octyn-3-one /1-decyn-3-one ;
- 1-(p-tolyl)-2-butyn-1-one /1-decyn-3-one ;
- 2,5-heptadiyn-4-one /1-decyn-3-one ;
- 1-(p-methoxyphenyl)-2-propyn-1-one / 1-decyn-3-one ;
- 1,4-diphenyl-2-butyn-1,4-dione / 1-decyn-3-one ;
- 4,7-undecadiyn-6-one / 1-decyn-3-one ;
- 1-(2-furyl)-2-octyn-1-one / 1-phenyl-2-propyn-1-one ;
- 1-(2-furyl)-3-phenyl-2-propyn-1-one / 1-phenyl-2-propyn-1-one ;
- 1-octyn-3-one / 1-phenyl-2-propyn-1-one ;
- 1-(p-tolyl)-2-butyn-1-one / 1-phenyl-2-propyn-1-one ;
- 2,5-heptadiyn-4-one / 1-phenyl-2-propyn-1-one ;
- 1-(p-methoxyphenyl)-2-propyn-1-one / 1-phenyl-2-propyn-1-one ;
- 1,4-diphenyl-2-butyn-1,4-dione / 1-phenyl-2-propyn-1-one ;
- 4,7-undecadiyn-6-one / 1-phenyl-2-propyn-1-one ;
- 1-(2-furyl)-3-phenyl-2-propyn-1-one / 1-(2-furyl)-2-octyn-1-one ;
- 1-octyn-3-one/ 1-(2-furyl)-2-octyn-1-one 1-(p-tolyl)-2-butyn-1-one ;
- 2,5-heptadiyn-4-one / 1-(2-furyl)-2-octyn-1-one ;
- 1-(p-methoxyphenyl)-2-propyn-1-one / 1-(2-furyl)-2-octyn-1-one
- 1,4-diphenyl-2-butyn-1,4-dione / 1-(2-furyl)-2-octyn-1-one ;
- 4,7-undecadiyn-6-one / 1-(2-furyl)-2-octyn-1-one ;
- 1-octyn-3-one / 1-(2-furyl)-3-phenyl-2-propyn-1-one ;
- 1-(p-tolyl)-2-butyn-1-one / 1-(2-furyl)-3-phenyl-2-propyn-1-one ;
- 2,5-heptadiyn-4-one / 1-(2-furyl)-3-phenyl-2-propyn-1-one ;
- 1-(p-methoxyphenyl)-2-propyn-1-one / 1-(2-furyl)-3-phenyl-2-propyn-1-one ;
- 1,4-diphenyl-2-butyn-1,4-dione / 1-(2-furyl)-3-phenyl-2-propyn-1-one ;
- 4,7-undecadiyn-6-one / 1-(2-furyl)-3-phenyl-2-propyn-1-one ;
- 1-(p-tolyl)-2-butyn-1-one / 1-octyn-3-one ;
- 2,5-heptadiyn-4-one / 1-octyn-3-one ;
- 1-(p-methoxyphenyl)-2-propyn-1-one / 1-octyn-3-one ;
- 1,4-diphenyl-2-butyn-1,4-dione / 1-octyn-3-one ;
- 4,7-undecadiyn-6-one / 1-octyn-3-one ;
- 2,5-heptadiyn-4-one / 1-(p-tolyl)-2-butyn-1-one ;
- 1-(p-methoxyphenyl)-2-propyn-1-one / 1-(p-tolyl)-2-butyn-1-one ;
- 1,4-diphenyl-2-butyn-1,4-dione / 1-(p-tolyl)-2-butyn-1-one ;
- 4,7-undecadiyn-6-one / 1-(p-tolyl)-2-butyn-1-one ;
- 1-(p-methoxyphenyl)-2-propyn-1-one / 2,5-heptadiyn-4-one ;
- 1,4-diphenyl-2-butyn-1,4-dione / 2,5-heptadiyn-4-one ;
- 4,7-undecadiyn-6-one / 2,5-heptadiyn-4-one ;
- 1,4-diphenyl-2-butyn-1,4-dione / 1-(p-methoxyphenyl)-2-propyn-1-one ;
- 4,7-undecadiyn-6-one / 1-(p-methoxyphenyl)-2-propyn-1-one ;
- 4,7-undecadiyn-6-one / 1,4-diphenyl-2-butyn-1,4-dione.

10. Composition selon l'une quelconque des revendications 8 ou 9, **caractérisé en ce qu'**elle comprend en outre au moins un aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels, les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique, et encore plus préférentiellement dans une composition désodorisante comprenant un mélange d'au moins un premier aldéhyde (aldéhyde de classe A) choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels et d'au moins un second aldéhyde (aldéhyde de classe B) choisi parmi les aldéhydes possédant une insaturation non aromatique portée par le carbone en alpha de la fonction aldéhyde, les aldéhydes possédant une insaturation en alpha de la fonction aldéhyde conjuguée avec un cycle aromatique et les aldéhydes dont la fonction est portée par un cycle aromatique.

## Patentansprüche

1. Verwendung, in einer Desodorisierungszusammensetzung, zum Zerstören der Übelgeruchsmoleküle von wenigstens einer Verbindung der Familie der α-Acetylenketone, umfassend wenigstens eine Kette -CO-C≡C- oder -C=C-CO- entsprechend der allgemeinen Formel 1
**R-(CO)ₖ-C≡C-(A)-(C≡C)ₘ-(CO)ₙ-R₁** **(1)**
wobei
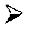 R und R₁ unabhängig oder gleichzeitig ein Radikal repräsentieren können, ausgewählt aus
∘ einer Alkylkette mit 1 bis 9, vorzugsweise 3 bis 7 Kohlenstoffatomen, linear oder verzweigt, substituiert oder nicht, oder
∘ einem Cycloalkan mit 3 bis 8, vorzugsweise 5 oder 6 Kohlenstoffatomen, substituiert oder nicht; oder
∘ einem Furanring, gesättigt oder nicht, substituiert oder nicht; oder
∘ einem Pyranring, gesättigt oder nicht, substituiert oder nicht; oder
∘ einem aromatischen Ring mit 6 bis 8 Kohlenstoffatomen, substituiert oder nicht; oder
∘ einem Wasserstoffatom;
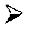 A eine Gruppe repräsentiert, ausgewählt aus -(CH₂)ₓ, wobei x eine ganze Zahl von 0 bis 6, vorzugsweise von 0 bis 4 repräsentiert, (-CO)ₗ, wobei l eine ganze Zahl von 0 oder 1 repräsentiert, oder einer Kette -(CH₂)_{y}-O-(CH₂)_{z}-, wobei y und z, gleichzeitig oder unabhängig, eine ganze Zahl von 0 bis 6, vorzugsweise von 0 bis 4 repräsentieren, wobei zu verstehen ist, dass x + y den Wert 6 nicht übersteigt;
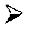 k, m und n, gleichzeitig oder unabhängig, eine ganze Zahl von 0 oder 1 sind, wobei zu verstehen ist, dass k und n nicht gleichzeitig 0 sein können, wenn l gleich 0 ist;
mit Ausnahme von 2-Methyl-4-nonyn-3-on von Formel C₄H₉-C≡C-CO-CH(CH₃)₂.

2. Verwendung nach Anspruch 1, **dadurch gekennzeichnet, dass** die Verbindung von Formel 1 ausgewählt ist aus:
- 1-Octyn-3-on von Formel C₅H₁₁-CO-C≡CH;
- 3-Octyn-2-on von Formel C₄H₉-C≡C-CO-CH₃;
- 4-Octyn-3-on von Formel C₃H₇C≡C-CO-C₂H₅;
- 4-Nonyn-3-on von Formel C₃H₇-CH₂-C≡C-CO-C₂H₅;
- 2-Nonyn-4-on von Formel CH₃-C≡C-CO-C₅H₁₁;
- 5-Nonyn-4-on von Formel C₃H₇-CO-C≡C-CH₂-C₂H₅;
- 1-Decyn-3-on von Formel C₇H₁₅-CO-C≡CH;
- 4-Decyn-3-on von Formel C₅H₁₁-C≡C-CO-C₂H₅;
- 6-Decyn-5-on von Formel C₃H₇-C≡C-CO-C₄H₉;
- 5-Decyn-4-on von Formel C₄H₉-C≡C-CO-C₃H₇;
- 1-(p-Methoxyphenyl)-2-propyn-1-on von Formel H₃C-O-C₆H₄-CO-C≡CH;
- 1-Cyclopropyl-2-heptyn-1-on von Formel C₄H₉-C≡C-CO-C₃H₅ (Cyclopropyl);
- 2-Methyl-5-decyn-4-on von Formel C₄H₉-C≡C-CO-CH₂-CH(CH₃)₂;
- 9-Methyl-5-decyn-4-on von Formel C₃H₇-CO-C≡C-CH₂-CH₂-CH(CH₃)₂;
- 2-Methyl-4-octyn-3-on von Formel C₃H₇-C≡C-CO-CH(CH₃)₂;
- 1-Phenyl-2-propyn-1-on von Formel C₆H₅-CO-C≡CH;
- 4-Phenyl-3-butyn-2-on von Formel C₆H₅-C≡C-CO-CH₃;
- 5-Phenyl-4-pentyn-3-on von Formel C₆H₅-C≡C-CO-C₂H₅;
- 1-Phenyl-1-heptyn-3-on von Formel C₆H₅-C≡C-CO-C₄H₉;
- 1-(2-Furyl)-3-phenyl-2-propyn-1-on von Formel C₆H₅-C≡C-CO-C₄H₃O (Furyl);
- 1-(2-Furyl)-2-octyn-1-on von Formel C₅H₁₁-C≡C-CO-C₄H₃O (Furyl);
- C₆H₅-C≡C-CO-C₄H₃O (Furyl);
- 3-Cyclopropyl-1-(p-tolyl)-2-propyn-1-on von Formel C₃H₂-C≡C-CO-C₆H₄-CH₃;
- 1-Cyclopropyl-4-methyl-1-hexyn-3-on von Formel C₃H₅-C≡C-CO-CH(C₂H₅)-CH₃ (Cyclopropyl);
- 9-Hexadecyn-8-on von Formel C₆H₁₃-C≡C-CO-C₇H₁₅;
- 5-Ethyl-11-methyl-7-dodecyn-6-on von Formel (CH₃)₂CH-C₂H₄-C≡C-CO-CH(C₂H₅)-C₄H₉;
- 7-Tetradecyn-6-on von Formel C₆H₁₃-C≡C-CO-C₅H₁₁;
- 1-Cyclohexyl-4-ethyl-1-hexyn-3-on von Formel C₆H₁₁-C≡C-CO-CH(C₂H₅)₂ (Cyclohexyl);
- 1-Cyclopentyl-4-nonyn-3-on von Formel C₄H₉-C≡C-CO-C₂H₄-C₅H₉ (Cyclopentyl);
- 1-Cyclohexyl-2-heptyn-1-on von Formel C₄H₉-C≡C-CO-C₆H₁₁ (Cyclohexyl);
- 1-(p-Tolyl)-2-butyn-1-on von Formel CH₃-C≡C-CO-C₆H₄-(p-CH₃);
- 2,2,8-Trimethyl-4-nonyn-3-on von Formel (CH₃)₂CH-C₂H₄-C≡C-CO-C(CH₃)₃;
- 2,2-Dimethyl-4-hexyn-3-on von Formel CH₃-C≡C-CO-C(CH₃)₃;
- 1,4-Diphenyl-2-butyn-1,4-dion von Formel C₆H₅-CO-C≡C-CO-C₆H₅;
- 5-Decyn-2,4-dion von Formel C₄H₉-C≡C-CO-CH₂-CO-CH₃;
- 1-(3,4-Dimethoxyphenyl)-2-butyn-1-on von Formel CH₃-C≡C-CO-C₆H₃-(OCH₃)₂;
- 2,5-Heptadiyn-4-on von Formel H₃C-C≡C-CO-C≡C-CH₃;
- 3-Hexyn-2,5-dion von Formel CH₃-CO-C≡C-CO-CH₃;
- 4-Octyn-3,6-dion von Formel C₂H₅-CO-C≡C-CO-C₂H₅;
- 2,7-Dimethyl-4-octyn-3,6-dion von Formel (CH₃)₂CH-CO-C≡C-CO-CH(CH₃)₂;
- 2,7-Dimethyl-4-octyn-3-on von Formel (CH₃)₂CH-CH₂-C≡C-CO-CH(CH₃)₂;
- 4,7-Undecadiyn-6-on von Formel C₃H₇-C≡C-CO-C≡C-C₃H₇;
- 5-Decyn-4,7-dion von Formel C₃H₇-CO-C≡C-CO-C₃H₇;
- 2,9-Dimethyl-5-decyn-4,7-dion von Formel (CH₃)₂CH-CH₂-CO-C≡C-CO-CH₂-CH(CH₃)₂;
- 6-Dodecyn-5,8-dion von Formel C₄H₉-CO-C≡C-CO-C₄H₉;
- 2,11-Dimethyl-6-dodecyn-5,8-dion von Formel (CH₃)₂CH-C₂H₄-CO-C≡C-CO-(CH₂)₂-CH(CH₃);
- 2,9-Dimethyl-5-decyn-4-on von Formel (CH₃)₂CH-C₂H₄-C≡C-CO-CH₂-CH(CH₃)₂;
- 7-Tetradecyn-6,9-dion von Formel C₅H₁₁-CO-C≡C-CO-C₅H₁₁;
- 4,10-Undecadiyn-3-on von Formel HC≡C-C₄H₈-C≡C-CO-C₂H₅;
- 2,12-Dimethyl-7-oxa-4,9-tridecadiyn-3,11-dion von Formel (CH₃)₂-CO-C≡C-CH₂-O-CH₂-C≡C-CO-CH(CH₃)₂, oder
- 4,10-Tetradecadiyn-3,12-dion von Formel C₂H₅-CO-C≡C-C₄H₈-C≡C-CO-C₂H₅.

3. Verwendung nach Anspruch 1 oder 2, **dadurch gekennzeichnet, dass** die Verbindung von Formel 1 ein Verbindungspaar von Formel 1 ist, ausgewählt aus den Paaren:
- 1-Phenyl-2-propyn-1-on / 1-Decyn-3-on;
- 1-(2-Furyl)-2-octyn-1-on / 1-Decyn-3-on;
- 1-(2-Furyl)-3-phenyl-2-propyn-1-on / 1-Decyn-3-on;
- 1-Octyn-3-on / 1-Decyn-3-on;
- 1-(p-Tolyl)-2-butyn-1-on / 1-Decyn-3-on;
- 2,5-Heptadiyn-4-on / 1-Decyn-3-on;
- 1-(p-Methoxyphenyl)-2-propyn-1-on / 1-Decyn-3-on;
- 1,4-Diphenyl-2-butyn-1,4-dion / 1-Decyn-3-on;
- 4,7-Undecadiyn-6-on / 1-Decyn-3-on;
- 1-(2-Furyl)-2-octyn-1-on / 1-Phenyl-2-propyn-1-on;
- 1-(2-Furyl)-3-phenyl-2-propyn-1-on / 1-Phenyl-2-propyn-1-on;
- 1-Octyn-3-on / 1-Phenyl-2-propyn-1-on;
- 1-(p-Tolyl)-2-butyn-1-on / 1-Phenyl-2-propyn-1-on;
- 2,5-Heptadiyn-4-on / 1-Phenyl-2-propyn-1-on;
- 1-(p-Methoxyphenyl)-2-propyn-1-on / 1-Phenyl-2-propyn-1-on;
- 1,4-Diphenyl-2-butyn-1,4-dion / 1-Phenyl-2-propyn-1-on;
- 4,7-Undecadiyn-6-on / 1-Phenyl-2-propyn-1-on;
- 1-(2-Furyl)-3-phenyl-2-propyn-1-on / 1-(2-Furyl)-2-octyn-1-on;
- 1-Octyn-3-on / 1-(2-Furyl)-2-octyn-1-on 1-(p-tolyl)-2-butyn-1-on;
- 2,5-Heptadiyn-4-on / 1-(2-Furyl)-2-octyn-1-on;
- 1-(p-Methoxyphenyl)-2-propyn-1-on / 1-(2-furyl)-2-octyn-1-on;
- 1,4-Diphenyl-2-butyn-1,4-dion / 1-(2-Furyl)-2-octyn-1-on;
- 4,7-Undecadiyn-6-on / 1-(2-Furyl)-2-octyn-1-on;
- 1-Octyn-3-on / 1-(2-Furyl)-3-phenyl-2-propyn-1-on;
- 1-(p-Tolyl)-2-butyn-1-on / 1-(2-Furyl)-3-phenyl-2-propyn-1-on;
- 2,5-Heptadiyn-4-on / 1-(2-Furyl)-3-phenyl-2-propyn-1-on;
- 1-(p-Methoxyphenyl)-2-propyn-1-on / 1-(2-Furyl)-3-phenyl-2-propyn-1-on;
- 1,4-Diphenyl-2-butyn-1,4-dion / 1-(2-Furyl)-3-phenyl-2-propyn-1-on;
- 4,7-Undecadiyn-6-on / 1-(2-Furyl)-3-phenyl-2-propyn-1-on;
- 1-(p-Tolyl)-2-butyn-1-on / 1-Octyn-3-on;
- 2,5-Heptadiyn-4-on / 1-Octyn-3-on;
- 1-(p-Methoxyphenyl)-2-propyn-1-on / 1-Octyn-3-on;
- 1,4-Diphenyl-2-butyn-1,4-dion / 1-Octyn-3-on;
- 4,7-Undecadiyn-6-on / 1-Octyn-3-on;
- 2,5-Heptadiyn-4-on / 1-(p-Tolyl)-2-butyn-1-on;
- 1-(p-Methoxyphenyl)-2-propyn-1-on / 1-(p-Tolyl)-2-butyn-1-on;
- 1,4-Diphenyl-2-butyn-1,4-dion / 1-(p-Tolyl)-2-butyn-1-on;
- 4,7-Undecadiyn-6-on / 1-(p-Tolyl)-2-butyn-1-on;
- 1-(p-Methoxyphenyl)-2-propyn-1-on / 2,5-Heptadiyn-4-on;
- 1,4-Diphenyl-2-butyn-1,4-dion / 2,5-Heptadiyn-4-on;
- 4,7-Undecadiyn-6-on / 2,5-Heptadiyn-4-on;
- 1,4-Diphenyl-2-butyn-1,4-dion / 1-(p-Methoxyphenyl)-2-propyn-1-on;
- 4,7-Undecadiyn-6-on / 1-(p-Methoxyphenyl)-2-propyn-1-on;
- 4,7-Undecadiyn-6-on / 1,4-Diphenyl-2-butyn-1,4-dion.

4. Verwendung nach einem der Ansprüche 1 bis 3, **dadurch gekennzeichnet, dass** die Verbindung von Formel 1 in einer Menge zwischen 0,1 % und 40 %, vorzugsweise zwischen 0,1 % und 10 % nach Gesamtgewicht der Zusammensetzung in der Zusammensetzung vorliegt.

5. Verwendung nach einem der Ansprüche 1 bis 4, **dadurch gekennzeichnet, dass** die Zusammensetzung ferner Folgendes umfasst: wenigstens ein Aldehyd, ausgewählt aus den aliphatischen acyclischen und Nicht-Terpenaldehyden, den alicyclischen Nicht-Terpenaldehyden, den Terpenaldehyden, den aliphatischen Aldehyden, substituiert durch eine aromatische Gruppe, den bifunktionellen Aldehyden, den Aldehyden, die eine nichtaromatische Ungesättigtheit besitzen, getragen von dem Kohlenstoff an der Alphaposition der Aldehydfunktion, den Aldehyden, die eine Ungesättigtheit in der Alphaposition der Aldehydfunktion besitzen, konjugiert mit einem aromatischen Ring und den Aldehyden, deren Funktion von einem aromatischen Ring getragen wird, und noch stärker bevorzugt in einer Desodorisierungszusammensetzung, die ein Gemisch aus wenigstens einem ersten Aldehyd umfasst, ausgewählt aus den aliphatischen acyclischen und Nicht-Terpenaldehyden, den alicyclischen Nicht-Terpenaldehyden, den Terpenaldehyden, den aliphatischen Aldehyden, substituiert durch eine aromatische Gruppe, und den bifunktionellen Aldehyden, und von wenigstens einem zweiten Aldehyd, ausgewählt aus den Aldehyden, die eine nicht aromatische Ungesättigtheit besitzen, getragen von dem Kohlenstoff in der Alphaposition der Aldehydfunktion, den Aldehyden, die eine Ungesättigtheit in der Alphaposition der Aldehydfunktion besitzen, konjugiert mit einem aromatischen Ring und den Aldehyden, deren Funktion von einem aromatischen Ring getragen wird.

6. Verwendung nach Anspruch 5, **dadurch gekennzeichnet, dass** sie eine Menge an Aldehyd zwischen 0,5 % und 50 % der Zusammensetzung, vorzugsweise eine Menge zwischen 1 % und 20 % der Zusammensetzung umfasst.

7. Verwendung nach Anspruch 5 oder 6, **dadurch gekennzeichnet, dass** die Aldehyde der Klassen A und B in relativen Anteilen mit Bezug zueinander von 80:20 bis 20:80, insbesondere in Anteilen von 50:50 und in einer Gesamtmenge von Aldehyden zwischen 1 % und 90 % der Zusammensetzung, vorzugsweise in einer Menge zwischen 10 % und 30 % der Zusammensetzung vorliegen.

8. Desodorisierungszusammensetzung, **dadurch gekennzeichnet, dass** sie wenigstens eine Verbindung der Familie der a-Acetylenketone entsprechend der allgemeinen Formel 1
**R-(CO)ₖ-C≡C-(A)-(C≡C)ₘ-(CO)ₙ-R₁** **(1)**
umfasst, ausgewählt aus:
- 1-Octyn-3-on von Formel C₅H₁₁-CO-C≡CH;
- 3-Octyn-2-on von Formel C₄H₉-C≡C-CO-CH₃;
- 4-Octyn 3-on von Formel C₃H₇-C≡C-CO-C₂H₅;
- 4-Nonyn-3-on von Formel C₃H₇-CH₂-C≡C-CO-C₂H₅;
- 2-Nonyn-4-on von Formel CH₃-C≡C-CO-C₅H₁₁,
- 5-Nonyn-4-on von Formel C₃H₇-CO-C≡C-CH₂-C₂H₅;
- 1-Decyn-3-on von Formel C₇H₁₅-CO-C≡CH;
- 4-Decyn-3-on von Formel C₅H₁₁-C≡C-CO-C₂H₅;
- 6-Decyn-5-on von Formel C₃H₇-C≡C-CO-C₄H₉;
- 5-Decyn-4-on von Formel C₄H₉-C≡C-CO-C₃H₇;
- 1-Cyclopropyl-2-heptyn-1-on von Formel C₄H₉-C≡C-CO-C₃H₅ (Cyclopropyl);
- 2-Methyl-5-decyn-4-on von Formel C₄H₉-C≡C-CO-CH₂-CH(CH₃)₂;
- 9-Methyl-5-decyn-4-on von Formel C₃H₇-CO-C≡C-CH₂-CH₂-CH(CH₃)₂;
- 2-Methyl-4-octyn-3-on von Formel C₃H₇-C≡C-CO-CH(CH₃)₂;
- 4-Phenyl-3-butyn-2-on von Formel C₆H₅-C≡C-CO-CH₃;
- 5-Phenyl-4-pentyn-3-on von Formel C₆H₅-C≡C-CO-C₂H₅;
- 1-Phenyl-1-heptyn-3-on von Formel C₆H₅-C≡C-CO-C₄H₉;
- 1-Cyclopropyl-4-methyl-1-hexyn-3-on von Formel C₃H₅-C≡C-CO-CH(C₂H₅)-CH₃ (Cyclopropyl);
- 9-Hexadecyn-8-on von Formel C₆H₁₃-C≡C-CO-C₇H₁₅;
- 5-Ethyl-11-methyl-7-dodecyn-6-on von Formel (CH₃)₂CH-C₂H₄-C≡C-CO-CH(C₂H₅)-C₄H₉;
- 7-Tetradecyn-6-on von Formel C₆H₁₃-C≡C-CO-C₅H₁₁;
- 1-Cyclohexyl-4-ethyl-1-hexyn-3-on von Formel C₆H₁₁-C≡C-CO-CH(C₂H₅)₂ (Cyclohexyl);
- 1-Cyclopentyl-4-nonyn-3-on von Formel C₄H₉-C≡C-CO-C₂H₄-C₅H₉ (Cyclopentyl);
- 1-Cyclohexyl-2-heptyn-1-on von Formel C₄H₉-C≡C-CO-C₆H₁₁ (Cyclohexyl);
- 2,2,8-Trimethyl-4-nonyn-3-on von Formel (CH₃)₂CH-C₂H₄-C≡C-CO-C(CH₃)₃;
- 2,2-Dimethyl-4-hexyn-3-on von Formel CH₃-C≡C-CO-C(CH₃)₃;
- 3-Hexyn-2,5-dion von Formel CH₃-CO-C≡C-CO-CH₃;
- 4-Octyn-3,6-dion von Formel C₂H₅-CO-C≡C-CO-C₂H₅;
- 2,7-Dimethyl-4-octyn-3,6-dion von Formel (CH₃)₂CH-CO-C≡C-CO-CH(CH₃)₂;
- 2,7-Dimethyl-4-octyn-3-on von Formel (CH₃)₂CH-CH₂-C≡C-CO-CH(CH₃)₂;
- 4,7-Undecadiyn-6-on von Formel C₃H₇-C≡C-CO-C≡C-C₃H₇;
- 5-Decyn-4,7-dion von Formel C₃H₇-CO-C≡C-CO-C₃H₇;
- 2,9-Dimethyl-5-decyn-4,7-dion von Formel (CH₃)₂CH-CH₂-CO-C≡C-CO-CH₂-CH(CH₃)₂;
- 2,11-Dimethyl-6-dodecyn-5,8-dion von Formel (CH₃)₂CH-C₂H₄-CO-C≡C-CO-(CH₂)₂-CH(CH₃);
- 2,9-Dimethyl-5-decyn-4-on von Formel (CH₃)₂CH-C₂H₄-C≡C-CO-CH₂-CH(CH₃)₂;
- 7-Tetradecyn-6,9-dion von Formel C₅H₁₁-CO-C≡C-CO-C₅H₁₁;
- 4,10-Undecadiyn-3-on von Formel HC≡C-C₄H₈-C≡C-CO-C₂H₅;
- 2,12-Dimethyl-7-oxa-4,9-tridecadiyn-3,11-dion von Formel (CH₃)₂-CO-C≡C-CH₂-O-CH₂-C≡C-CO-CH(CH₃)₂, oder
- 4,10-Tetradecadiyn-3,12-dion von Formel C₂H₅-CO-C≡C-C₄H₈-C≡C-CO-C₂H₅.

9. Desodorisierungszusammensetzung, **dadurch gekennzeichnet, dass** sie wenigstens eine Verbindung von Formel 1:
**R-(CO)ₖ-C≡C-(A)-(C≡C)ₘ-(CO)ₙ-R₁** **(1)**
umfasst, ausgewählt aus den Paaren:
- 1-Phenyl-2-propyn-1-on / 1-Decyn-3-on;
- 1-(2-Furyl)-2-octyn-1-on / 1-Decyn-3-on;
- 1-(2-Furyl)-3-phenyl-2-propyn-1-on / 1-Decyn-3-on;
- 1-Octyn-3-on / 1-Decyn-3-on;
- 1-(p-Tolyl)-2-butyn-1-on / 1-Decyn-3-on;
- 2,5-Heptadiyn-4-on / 1-Decyn-3-on;
- 1-(p-Methoxyphenyl)-2-propyn-1-on / 1-Decyn-3-on;
- 1,4-Diphenyl-2-butyn-1,4-dion / 1-Decyn-3-on;
- 4,7-Undecadiyn-6-on / 1-Decyn-3-on;
- 1-(2-Furyl)-2-octyn-1-on / 1-Phenyl-2-propyn-1-on;
- 1-(2-Furyl)-3-phenyl-2-propyn-1-on / 1-Phenyl-2-propyn-1-on;
- 1-Octyn-3-on / 1-Phenyl-2-propyn-1-on;
- 1-(p-Tolyl)-2-butyn-1-on / 1-Phenyl-2-propyn-1-on;
- 2,5-Heptadiyn-4-on / 1-Phenyl-2-propyn-1-on;
- 1-(p-Methoxyphenyl)-2-propyn-1-on / 1-Phenyl-2-propyn-1-on;
- 1,4-Diphenyl-2-butyn-1,4-dion / 1-Phenyl-2-propyn-1-on;
- 4,7-Undecadiyn-6-on / 1-Phenyl-2-propyn-1-on;
- 1-(2-Furyl)-3-phenyl-2-propyn-1-on / 1-(2-Furyl)-2-octyn-1-on;
- 1-Octyn-3-on / -(2-Furyl)-2-octyn-1-on 1-(p-tolyl)-2-butyn-1-on;
- 2,5-Heptadiyn-4-on / 1-(2-Furyl)-2-octyn-1-on;
- 1-(p-Methoxyphenyl)-2-propyn-1-on / 1-(2-Furyl)-2-octyn-1-on
- 1,4-Diphenyl-2-butyn-1,4-dion / 1-(2-Furyl)-2-octyn-1-on;
- 4,7-Undecadiyn-6-on / 1-(2-Furyl)-2-octyn-1-on;
- 1-Octyn-3-on / 1-(2-Furyl)-3-phenyl-2-propyn-1-on;
- 1-(p-Tolyl)-2-butyn-1-on / 1-(2-Furyl)-3-phenyl-2-propyn-1-on;
- 2,5-Heptadiyn-4-on / 1-(2-Furyl)-3-phenyl-2-propyn-1-on;
- 1-(p-Methoxyphenyl)-2-propyn-1-on / 1-(2-Furyl)-3-phenyl-2-propyn-1-on;
- 1,4-Diphenyl-2-butyn-1,4-dion / 1-(2-Furyl)-3-phenyl-2-propyn-1-on;
- 4,7-Undecadiyn-6-on / 1-(2-Furyl)-3-phenyl-2-propyn-1-on;
- 1-(p-Tolyl)-2-butyn-1-on / 1-Octyn-3-on;
- 2,5-Heptadiyn-4-on / 1-Octyn-3-on;
- 1-(p-Methoxyphenyl)-2-propyn-1-on / 1-Octyn-3-on;
- 1,4-Diphenyl-2-butyn-1,4-dion / 1-Octyn-3-on;
- 4,7-Undecadiyn-6-on / 1-Octyn-3-on;
- 2,5-Heptadiyn-4-on / 1-(p-Tolyl)-2-butyn-1-on;
- 1-(p-Methoxyphenyl)-2-propyn-1-on / 1-(p-Tolyl)-2-butyn-1-on;
- 1,4-Diphenyl-2-butyn-1,4-dion / 1-(p-Tolyl)-2-butyn-1-on;
- 4,7-Undecadiyn-6-on / 1-(p-Tolyl)-2-butyn-1-on;
- 1-(p-Methoxyphenyl)-2-propyn-1-on / 2,5-Heptadiyn-4-on;
- 1,4-Diphenyl-2-butyn-1,4-dion / 2,5-Heptadiyn-4-on;
- 4,7-Undecadiyn-6-on / 2,5-Heptadiyn-4-on;
- 1,4-Diphenyl-2-butyn-1,4-dion / 1-(p-Methoxyphenyl)-2-propyn-1-on;
- 4,7-Undecadiyn-6-on / 1-(p-Methoxyphenyl)-2-propyn-1-on;
- 4,7-Undecadiyn-6-on / 1,4-Diphenyl-2-butyn-1,4-dion.

10. Zusammensetzung nach Anspruch 8 oder 9, **dadurch gekennzeichnet, dass** sie ferner Folgendes umfasst: wenigstens ein Aldehyd, ausgewählt aus den aliphatischen acyclischen und Nicht-Terpenaldehyden, den alicyclischen Nicht-Terpenaldehyden, den Terpenaldehyden, den aliphatischen Aldehyden, substituiert durch eine aromatische Gruppe, den bifunktionellen Aldehyden, den Aldehyden, die eine nichtaromatische Ungesättigtheit besitzen, getragen von dem Kohlenstoff an der Alphaposition der Aldehydfunktion, den Aldehyden, die eine Ungesättigtheit in der Alphaposition der Aldehydfunktion besitzen, konjugiert mit einem aromatischen Ring und den Aldehyden, deren Funktion von einem aromatischen Ring getragen wird, und noch stärker bevorzugt in einer Desodorisierungszusammensetzung, die ein Gemisch aus wenigstens einem ersten Aldehyd (Aldehyd der Klasse A), ausgewählt aus den aliphatischen acyclischen und Nicht-Terpenaldehyden, den alicyclischen Nicht-Terpenaldehyden, den Terpenaldehyden, den aliphatischen Aldehyden, substituiert durch eine aromatische Gruppe, und den bifunktionellen Aldehyden, und wenigstens einem zweiten Aldehyd (Aldehyd der Klasse B), ausgewählt aus den Aldehyden, die eine nicht aromatische Ungesättigtheit besitzen, getragen von dem Kohlenstoff an der Alphaposition der Aldehydfunktion, den Aldehyden, die eine Ungesättigtheit an der Alphaposition der Aldehydfunktion besitzen, konjugiert mit einem aromatischen Ring und den Aldehyden, deren Funktion von einem aromatischen Ring getragen wird, umfasst.

## Claims

1. Use, in a deodorant composition, for destroying malodorous molecules of at least one compound of the family of the α-acetylenic ketones comprising at least one -CO-C≡C- or -C=C-CO- chain formation, corresponding to general formula 1
**R-(CO)ₖ-C≡C-(A)-(C≡C)ₘ-(CO)ₙ-R₁** **(1)**
in which
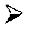 R and R₁ can represent, independently or simultaneously, a radical selected from
∘ an alkyl chain comprising from 1 to 9, preferably from 3 to 7 carbon atoms, linear or branched, substituted or unsubstituted, or
∘ a cycloalkane comprising from 3 to 8, preferably 5 or 6 carbon atoms, substituted or unsubstituted; or
∘ a furan ring, saturated or unsaturated, substituted or unsubstituted; or
∘ a pyran ring, saturated or unsaturated, substituted or unsubstituted; or
∘ an aromatic ring comprising from 6 to 8 carbon atoms, substituted or unsubstituted; or also
∘ a hydrogen atom;
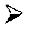 A represents a group selected from -(CH₂)ₓ with x representing an integer with a value from 0 to 6, preferably from 0 to 4, (-CO)ₗ with l representing an integer with a value of 0 or 1, or also a -(CH₂)_{y}-O-(CH₂)_{z}- chain formation with y and z representing, simultaneously or independently, an integer with a value from 0 to 6, preferably from 0 to 4, it being understood that x + y does not exceed the value 6;
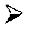 k, m and n are, simultaneously or independently, an integer with a value of 0 or 1, it being understood that k and n cannot simultaneously have a value of 0 if l is equal to 0;
with the exception of 2-methyl-4-nonyn-3-one of formula C₄H₉-C≡C-CO-CH(CH₃)₂.

2. Use according to claim 1, **characterized in that** the compound of formula 1 is selected from
- 1-octyn-3-one of formula C₅H₁₁-CO-C≡CH;
- 3-octyn-2-one of formula C₄H₉-C≡C-CO-CH₃;
- 4-octyn 3-one of formula C₃H₇-C≡C-CO-C₂H₅;
- 4-nonyn-3-one of formula C₃H₇-CH₂-C≡C-CO-C₂H₅;
- 2-nonyn-4-one of formula CH₃-C≡C-CO-C₅H₁₁;
- 5-nonyn-4-one of formula C₃H₇-CO-C≡C-CH₂-C₂H₅;
- 1-decyn-3-one of formula C₇H₁₅-CO-C≡CH;
- 4-decyn-3-one of formula C₅H₁₁-C≡C-CO-C₂H₅;
- 6-decyn-5-one of formula C₃H₇-C≡C-CO-C₄H₉;
- 5-decyn-4-one of formula C₄H₉-C≡C-CO-C₃H₇;
- 1-(p-methoxyphenyl)-2-propyn-1-one of formula H₃C-O-C₆H₄-CO-C≡CH;
- 1-cyclopropyl-2-heptyn-1-one of formula C₄H₉-C≡C-CO-C₃H₅ (cyclopropyl);
- 2-methyl-5-decyn-4-one of formula C₄H₉-C≡C-CO-CH₂-CH(CH₃)₂;
- 9-methyl-5-decyn-4-one of formula C₃H₇-CO-C≡C-CH₂-CH₂-CH(CH₃)₂;
- 2-methyl-4-octyn-3-one of formula C₃H₇-C≡C-CO-CH(CH₃)₂;
- 1-phenyl-2-propyn-1-one of formula C₆H₅-CO-C≡CH;
- 4-phenyl-3-butyn-2-one of formula C₆H₅-C≡C-CO-CH₃;
- 5-phenyl-4-pentyn-3-one of formula C₆H₅-C≡C-CO-C₂H₅;
- 1-phenyl-1-heptyn-3-one of formula C₆H₅-C≡C-CO-C₄H₉;
- 1-(2-furyl)-3-phenyl-2-propyn-1-one of formula C₆H₅-C≡C-CO-C₄H₃O (furyl);
- 1-(2-furyl)-2-octyn-1-one of formula C₅H₁₁-C≡C-CO-C₄H₃O (furyl);
- C₆H₅-C≡C-CO-C₄H₃O (furyl);
- 3-cyclopropyl-1-(p-tolyl)-2-propyn-1-one of formula C₃H₂-C≡C-CO-C₆H₄-CH₃;
- 1-cyclopropyl-4-methyl-1-hexyn-3-one of formula C₃H₅-C≡C-CO-CH(C₂H₅)-CH₃ (cyclopropyl);
- 9-hexadecyn-8-one of formula C₆H₁₃-C≡C-CO-C₇H₁₅;
- 5-ethyl-11-methyl-7-dodecyn-6-one of formula (CH₃)₂CH-C₂H₄-C≡C-CO-CH(C₂H₅)-C₄H₉;
- 7-tetradecyn-6-one of formula C₆H₁₃-C≡C-CO-C₅H₁₁;
- 1-cyclohexyl-4-ethyl-1-hexyn-3-one of formula C₆H₁₁-C≡C-CO-CH(C₂H₅)₂ (cyclohexyl);
- 1-cyclopentyl-4-nonyn-3-one of formula C₄H₉-C≡C-CO-C₂H₄-C₅H₉ (cyclopentyl);
- 1-cyclohexyl-2-heptyn-1-one of formula C₄H₉-C≡C-CO-C₆H₁₁ (cyclohexyl);
- 1-(p-tolyl)-2-butyn-1-one of formula CH₃-C≡C-CO-C₆H₄-(p-CH₃);
- 2,2,8-trimethyl-4-nonyn-3-one of formula (CH₃)₂CH-C₂H₄-C≡C-CO-C(CH₃)₃;
- 2,2-dimethyl-4-hexyn-3-one of formula CH₃-C≡C-CO-C(CH₃)₃;
- 1,4-diphenyl-2-butyn-1,4-dione of formula C₆H₅-CO-C≡C-CO-C₆H₅;
- 5-decyn-2,4-dione of formula C₄H₉-C≡C-CO-CH₂-CO-CH₃;
- 1-(3,4-dimethoxyphenyl)-2-butyn-1-one of formula CH₃-C≡C-CO-C₆H₃-(OCH₃)₂;
- 2,5-heptadiyn-4-one of formula H₃C-C≡C-CO-C≡C-CH₃;
- 3-hexyn-2,5-dione of formula CH₃-CO-C≡C-CO-CH₃;
- 4-octyn-3,6-dione of formula C₂H₅-CO-C≡C-CO-C₂H₅;
- 2,7-dimethyl-4-octyn-3,6-dione of formula (CH₃)₂CH-CO-C≡C-CO-CH(CH₃)₂;
- 2,7-dimethyl-4-octyn-3-one of formula (CH₃)₂CH-CH₂-C≡C-CO-CH(CH₃)₂;
- 4,7-undecadiyn-6-one of formula C₃H₇-C≡C-CO-C≡C-C₃H₇;
- 5-decyn-4,7-dione of formula C₃H₇-CO-C≡C-CO-C₃H₇;
- 2,9-dimethyl-5-decyn-4,7-dione of formula (CH₃)₂CH-CH₂-CO-C≡C-CO-CH₂-CH(CH₃)₂;
- 6-dodecyn-5,8-dione of formula C₄H₉-CO-C≡C-CO-C₄H₉;
- 2,11-dimethyl-6-dodecyn-5,8-dione of formula (CH₃)₂CH-C₂H₄-CO-C≡C-CO-(CH₂)₂-CH(CH₃);
- 2,9-dimethyl-5-decyn-4-one of formula (CH₃)₂CH-C₂H₄-C≡C-CO-CH₂-CH(CH₃)₂;
- 7-tetradecyn-6,9-dione of formula C₅H₁₁-CO-C≡C-CO-C₅H₁₁;
- 4,10-undecadiyn-3-one of formula HC≡C-C₄H₈-C≡C-CO-C₂H₅;
- 2,12-dimethyl-7-oxa-4,9-tridecadiyn-3,11-dione of formula (CH₃)₂-CO-C≡C-CH₂-O-CH₂-C≡C-CO-CH(CH₃)₂; or also
- 4,10-tetradecadiyn-3,12-dione of formula C₂H₅-CO-C≡C-C₄H₈-C≡C-CO-C₂H₅.

3. Use according to any one of claims 1 or 2, **characterized in that** the compound of formula 1 is a pair of compounds of formula 1 selected from the pairs
- 1-phenyl-2-propyn-1-one / 1-decyn-3-one;
- 1-(2-furyl)-2-octyn-1-one / 1-decyn-3-one;
- 1-(2-furyl)-3-phenyl-2-propyn-1-one / 1-decyn-3-one;
- 1-octyn-3-one / 1-decyn-3-one;
- 1-(p-tolyl)-2-butyn-1-one / 1-decyn-3-one;
- 2,5-heptadiyn-4-one / 1-decyn-3-one;
- 1-(p-methoxyphenyl)-2-propyn-1-one / 1-decyn-3-one;
- 1,4-diphenyl-2-butyn-1,4-dione / 1-decyn-3-one;
- 4,7-undecadiyn-6-one / 1-decyn-3-one;
- 1-(2-furyl)-2-octyn-1-one / 1-phenyl-2-propyn-1-one;
- 1-(2-furyl)-3-phenyl-2-propyn-1-one / 1-phenyl-2-propyn-1-one;
- 1-octyn-3-one / 1-phenyl-2-propyn-1-one;
- 1-(p-tolyl)-2-butyn-1-one / 1-phenyl-2-propyn-1-one;
- 2,5-heptadiyn-4-one / 1-phenyl-2-propyn-1-one;
- 1-(p-methoxyphenyl)-2-propyn-1-one / 1-phenyl-2-propyn-1-one;
- 1,4-diphenyl-2-butyn-1,4-dione / 1-phenyl-2-propyn-1-one;
- 4,7-undecadiyn-6-one / 1-phenyl-2-propyn-1-one;
- 1-(2-furyl)-3-phenyl-2-propyn-1-one / 1-(2-furyl)-2-octyn-1-one;
- 1-octyn-3-one / 1-(2-furyl)-2-octyn-1-one 1-(p-tolyl)-2-butyn-1-one;
- 2,5-heptadiyn-4-one / 1-(2-furyl)-2-octyn-1-one;
- 1-(p-methoxyphenyl)-2-propyn-1-one / 1-(2-furyl)-2-octyn-1-one;
- 1,4-diphenyl-2-butyn-1,4-dione / 1-(2-furyl)-2-octyn-1-one;
- 4,7-undecadiyn-6-one / 1-(2-furyl)-2-octyn-1-one;
- 1-octyn-3-one / 1-(2-furyl)-3-phenyl-2-propyn-1-one;
- 1-(p-tolyl)-2-butyn-1-one / 1-(2-furyl)-3-phenyl-2-propyn-1-one;
- 2,5-heptadiyn-4-one / 1-(2-furyl)-3-phenyl-2-propyn-1-one;
- 1-(p-methoxyphenyl)-2-propyn-1-one / 1-(2-furyl)-3-phenyl-2-propyn-1-one;
- 1,4-diphenyl-2-butyn-1,4-dione / 1-(2-furyl)-3-phenyl-2-propyn-1-one;
- 4,7-undecadiyn-6-one / 1-(2-furyl)-3-phenyl-2-propyn-1-one;
- 1-(p-tolyl)-2-butyn-1-one / 1-octyn-3-one;
- 2,5-heptadiyn-4-one / 1-octyn-3-one;
- 1-(p-methoxyphenyl)-2-propyn-1-one / 1-octyn-3-one;
- 1,4-diphenyl-2-butyn-1,4-dione / 1-octyn-3-one;
- 4,7-undecadiyn-6-one / 1-octyn-3-one;
- 2,5-heptadiyn-4-one / 1-(p-tolyl)-2-butyn-1-one;
- 1-(p-methoxyphenyl)-2-propyn-1-one / 1-(p-tolyl)-2-butyn-1-one;
- 1,4-diphenyl-2-butyn-1,4-dione / 1-(p-tolyl)-2-butyn-1-one;
- 4,7-undecadiyn-6-one / 1-(p-tolyl)-2-butyn-1-one;
- 1-(p-methoxyphenyl)-2-propyn-1-one / 2,5-heptadiyn-4-one;
- 1,4-diphenyl-2-butyn-1,4-dione / 2,5-heptadiyn-4-one;
- 4,7-undecadiyn-6-one / 2,5-heptadiyn-4-one;
- 1,4-diphenyl-2-butyn-1,4-dione / 1-(p-methoxyphenyl)-2-propyn-1-one;
- 4,7-undecadiyn-6-one / 1-(p-methoxyphenyl)-2-propyn-1-one;
- 4,7-undecadiyn-6-one / 1,4-diphenyl-2-butyn-1,4-dione.

4. Use according to any one of claims 1 to 3, **characterized in that** the compound of formula 1 is in the composition in a quantity comprised between 0.1 % and 40%, preferably between 0.1 % and 10% of the total weight of the composition.

5. Use according to any one of claims 1 to 4, **characterized in that** the composition further comprises at least one aldehyde selected from the acyclic and non-terpenic aliphatic aldehydes, the non-terpenic alicyclic aldehydes, the terpenic aldehydes, the aliphatic aldehydes substituted by an aromatic group, the bifunctional aldehydes, the aldehydes having a non-aromatic unsaturation borne by the carbon in the alpha position of the aldehyde function, the aldehydes having an unsaturation in the alpha position of the aldehyde function conjugated with an aromatic ring and the aldehydes the function of which is borne by an aromatic ring, and even more preferably in a deodorant composition comprising a mixture of at least one first aldehyde selected from the acyclic and non-terpenic aliphatic aldehydes, the non-terpenic alicyclic aldehydes, the terpenic aldehydes, the aliphatic aldehydes substituted by an aromatic group and the bifunctional aldehydes and at least one second aldehyde selected from the aldehydes having a non-aromatic unsaturation borne by the carbon in the alpha position of the aldehyde function, the aldehydes having an unsaturation in the alpha position of the aldehyde function conjugated with an aromatic ring and the aldehydes the function of which is borne by an aromatic ring.

6. Use according to claim 5, **characterized in that** it comprises a quantity of the aldehyde comprised between 0.5% and 50% of the composition, preferably in a quantity comprised between 1% and 20% of the composition.

7. Use according to any one of claims 5 or 6, **characterized in that** the aldehydes of classes A and B are in proportions relative to each other in proportions from 80/20 to 20/80, in particular in proportions of 50/50 and in a total quantity of aldehydes comprised between 1% and 90% of the composition, preferably in a quantity comprised between 10% and 30% of the composition.

8. Deodorant composition **characterized in that** it comprises at least one compound of the family of the α-acetylenic ketones corresponding to general formula 1
**R-(CO)k-C≡C-(A)-(C≡C)m-(CO)n-R₁** **(1)**
selected from
- 1-octyn-3-one of formula C₅H₁₁-CO-C≡CH;
- 3-octyn-2-one of formula C₄H₉-C≡C-CO-CH₃;
- 4-octyn 3-one of formula C₃H₇-C≡C-CO-C₂H₅;
- 4-nonyn-3-one of formula C₃H₇-CH₂-C≡C-CO-C₂H₅;
- 2-nonyn-4-one of formula CH₃-C≡C-CO-C₅H₁₁;
- 5-nonyn-4-one of formula C₃H₇-CO-C≡C-CH₂-C₂H₅;
- 1-decyn-3-one of formula C₇H₁₅-CO-C≡CH;
- 4-decyn-3-one of formula C₅H₁₁-C≡C-CO-C₂H₅;
- 6-decyn-5-one of formula C₃H₇-C≡C-CO-C₄H₉;
- 5-decyn-4-one of formula C₄H₉-C≡C-CO-C₃H₇;
- 1-cyclopropyl-2-heptyn-1-one of formula C₄H₉-C≡C-CO-C₃H₅ (cyclopropyl);
- 2-methyl-5-decyn-4-one of formula C₄H₉-C≡C-CO-CH₂-CH(CH₃)₂;
- 9-methyl-5-decyn-4-one of formula C₃H₇-CO-C≡C-CH₂-CH₂-CH(CH₃)₂;
- 2-methyl-4-octyn-3-one of formula C₃H₇-C≡C-CO-CH(CH₃)₂;
- 4-phenyl-3-butyn-2-one of formula C₆H₅-C≡C-CO-CH₃;
- 5-phenyl-4-pentyn-3-one of formula C₆H₅-C≡C-CO-C₂H₅;
- 1-phenyl-1-heptyn-3-one of formula C₆H₅-C≡C-CO-C₄H₉;
- 1-cyclopropyl-4-methyl-1-hexyn-3-one of formula C₃H₅-C≡C-CO-CH(C₂H₅)-CH₃ (cyclopropyl);
- 9-hexadecyn-8-one of formula C₆H₁₃-C=C-CO-C₇H₁₅;
- 5-ethyl-11-methyl-7-dodecyn-6-one of formula (CH₃)₂CH-C₂H₄-C=C-CO-CH(C₂H₅)-C₄H₉;
- 7-tetradecyn-6-one of formula C₆H₁₃-C≡C-CO-C₅H₁₁;
- 1-cyclohexyl-4-ethyl-1-hexyn-3-one of formula C₆H₁₁-C≡C-CO-CH(C₂H₅)₂ (cyclohexyl);
- 1-cyclopentyl-4-nonyn-3-one of formula C₄H₉-C≡C-CO-C₂H₄-C₅H₉ (cyclopentyl);
- 1-cyclohexyl-2-heptyn-1-one of formula C₄H₉-C≡C-CO-C₆H₁₁ (cyclohexyl);
- 2,2,8-trimethyl-4-nonyn-3-one of formula (CH₃)₂CH-C₂H₄-C≡C-CO-C(CH₃)₃;
- 2,2-dimethyl-4-hexyn-3-one of formula CH₃-C≡C-CO-C(CH₃)₃;
- 3-hexyn-2,5-dione of formula CH₃-CO-C≡C-CO-CH₃;
- 4-octyn-3,6-dione of formula C₂H₅-CO-C≡C-CO-C₂H₅;
- 2,7-dimethyl-4-octyn-3,6-dione of formula (CH₃)₂CH-CO-C≡C-CO-CH(CH₃)₂;
- 2,7-dimethyl-4-octyn-3-one of formula (CH₃)₂CH-CH₂-C≡C-CO-CH(CH₃)₂;
- 4,7-undecadiyn-6-one of formula C₃H₇-C≡C-CO-C≡C-C₃H₇;
- 5-decyn-4,7-dione of formula C₃H₇-CO-C≡C-CO-C₃H₇;
- 2,9-dimethyl-5-decyn-4,7-dione of formula (CH₃)₂CH-CH₂-CO-C=C-CO-CH₂-CH(CH₃)₂;
- 2,11-dimethyl-6-dodecyn-5,8-dione of formula (CH₃)₂CH-C₂H₄-CO-C≡C-CO-(CH₂)₂-CH(CH₃);
- 2,9-dimethyl-5-decyn-4-one of formula (CH₃)₂CH-C₂H₄-C≡C-CO-CH₂-CH(CH₃)₂;
- 7-tetradecyn-6,9-dione of formula C₅H₁₁-CO-C≡C-CO-C₅H₁₁;
- 4,10-undecadiyn-3-one of formula HC≡C-C₄H₈-C=C-CO-C₂H₅;
- 2,12-dimethyl-7-oxa-4,9-tridecadiyn-3,11-dione of formula (CH₃)₂-CO-C≡C-CH₂-O-CH₂-C≡C-CO-CH(CH₃)₂; or also
- 4,10-tetradecadiyn-3,12-dione of formula C₂H₅-CO-C≡C-C₄H₈-C≡C-CO-C₂H₅.

9. Deodorant composition **characterized in that** it comprises at least one compound of formula 1
**R-(CO)ₖ-C=C-(A)-(C≡C)m-(CO)ₙ-R₁** **(1)**
selected from the pairs
- 1-phenyl-2-propyn-1-one / 1-decyn-3-one;
- 1-(2-furyl)-2-octyn-1-one / 1-decyn-3-one;
- 1-(2-furyl)-3-phenyl-2-propyn-1-one / 1-decyn-3-one;
- 1-octyn-3-one / 1-decyn-3-one;
- 1-(p-tolyl)-2-butyn-1-one / 1-decyn-3-one;
- 2,5-heptadiyn-4-one / 1-decyn-3-one;
- 1-(p-methoxyphenyl)-2-propyn-1-one / 1-decyn-3-one;
- 1,4-diphenyl-2-butyn-1,4-dione / 1-decyn-3-one;
- 4,7-undecadiyn-6-one / 1-decyn-3-one;
- 1-(2-furyl)-2-octyn-1-one / -phenyl-2-propyn-1-one;
- 1-(2-furyl)-3-phenyl-2-propyn-1-one / -phenyl-2-propyn-1-one;
- 1-octyn-3-one / 1-phenyl-2-propyn-1-one;
- 1-(p-tolyl)-2-butyn-1-one / 1-phenyl-2-propyn-1-one;
- 2,5-heptadiyn-4-one / 1-phenyl-2-propyn-1-one;
- 1-(p-methoxyphenyl)-2-propyn-1-one / 1-phenyl-2-propyn-1-one;
- 1,4-diphenyl-2-butyn-1,4-dione / 1-phenyl-2-propyn-1-one;
- 4,7-undecadiyn-6-one / 1-phenyl-2-propyn-1-one;
- 1-(2-furyl)-3-phenyl-2-propyn-1-one / 1-(2-furyl)-2-octyn-1-one;
- 1-octyn-3-one / -(2-fury!)-2-octyn-1 -one 1-(p-tolyl)-2-butyn-1-one;
- 2,5-heptadiyn-4-one / 1-(2-furyl)-2-octyn-1-one;
- 1-(p-methoxyphenyl)-2-propyn-1-one / 1-(2-furyl)-2-octyn-1-one;
- 1,4-diphenyl-2-butyn-1,4-dione / 1-(2-furyl)-2-octyn-1-one;
- 4,7-undecadiyn-6-one / 1-(2-furyl)-2-octyn-1-one;
- 1-octyn-3-one / 1-(2-furyl)-3-phenyl-2-propyn-1-one;
- 1-(p-tolyl)-2-butyn-1-one / 1-(2-furyl)-3-phenyl-2-propyn-1-one;
- 2,5-heptadiyn-4-one / 1-(2-furyl)-3-phenyl-2-propyn-1-one;
- 1 -(p-methoxyphenyl)-2-propyn-1 -one 1-(2-furyl)-3-phenyl-2-propyn-1-one;
- 1,4-diphenyl-2-butyn-1,4-dione / 1-(2-furyl)-3-phenyl-2-propyn-1-one;
- 4,7-undecadiyn-6-one / 1-(2-furyl)-3-phenyl-2-propyn-1-one;
- 1-(p-tolyl)-2-butyn-1-one / 1-octyn-3-one;
- 2,5-heptadiyn-4-one / 1-octyn-3-one;
- 1-(p-methoxyphenyl)-2-propyn-1-one / 1-octyn-3-one;
- 1,4-diphenyl-2-butyn-1,4-dione / 1-octyn-3-one;
- 4,7-undecadiyn-6-one / 1-octyn-3-one;
- 2,5-heptadiyn-4-one / 1-(p-tolyl)-2-butyn-1-one;
- 1-(p-methoxyphenyl)-2-propyn-1-one / 1-(p-tolyl)-2-butyn-1-one;
- 1,4-diphenyl-2-butyn-1,4-dione / 1-(p-tolyl)-2-butyn-1-one;
- 4,7-undecadiyn-6-one / 1-(p-tolyl)-2-butyn-1-one;
- 1-(p-methoxyphenyl)-2-propyn-1-one / 2,5-heptadiyn-4-one;
- 1,4-diphenyl-2-butyn-1,4-dione / 2,5-heptadiyn-4-one;
- 4,7-undecadiyn-6-one / 2,5-heptadiyn-4-one;
- 1,4-diphenyl-2-butyn-1,4-dione / 1-(p-methoxyphenyl)-2-propyn-1-one;
- 4,7-undecadiyn-6-one / 1-(p-methoxyphenyl)-2-propyn-1-one;
- 4,7-undecadiyn-6-one / 1,4-diphenyl-2-butyn-1,4-dione.

10. Composition according to any one of claims 8 or 9, **characterized in that** it further comprises at least one aldehyde selected from the acyclic and non-terpenic aliphatic aldehydes, the non-terpenic alicyclic aldehydes, the terpenic aldehydes, the aliphatic aldehydes substituted by an aromatic group, the bifunctional aldehydes, the aldehydes having a non-aromatic unsaturation borne by the carbon in the alpha position of the aldehyde function, the aldehydes having an unsaturation in the alpha position of the aldehyde function conjugated with an aromatic ring and the aldehydes the function of which is borne by an aromatic ring, and even more preferably in a deodorant composition comprising a mixture of at least one first aldehyde (aldehyde of class A) selected from the acyclic and non-terpenic aliphatic aldehydes, the non-terpenic alicyclic aldehydes, the terpenic aldehydes, the aliphatic aldehydes substituted by an aromatic group and the bifunctional aldehydes and at least one second aldehyde (aldehyde of class B) selected from the aldehydes having a non-aromatic unsaturation borne by the carbon in the alpha position of the aldehyde function, the aldehydes having an unsaturation in the alpha position of the aldehyde function conjugated with an aromatic ring and the aldehydes the function of which is borne by an aromatic ring.
